# EUROPEAN PATENT SPECIFICATION

(11) **EP 4 537 855 B1**
(45) Date of publication and mention of the grant of the patent: **11.11.2026**
(21) Application number: 24171280.1
(22) Date of filing: 19.04.2024
(51) Int. Cl.: A61K 47/69

(54) **NANOPARTICLE FOR USE IN A PROPHYLAXIS OR IN A TREATMENT OF A CALCIFICATION OF BRUCH'S MEMBRANE**
NANOPARTIKEL ZUR VERWENDUNG IN EINER PROPHYLAXE ODER IN EINER BEHANDLUNG EINER VERKALKUNG DER BRUCH'SCHEN-MEMBRAN
NANOPARTICULE DESTINÉE À ÊTRE UTILISÉE DANS UNE PROPHYLAXIE OU UN TRAITEMENT D'UNE CALCIFICATION DE LA MEMBRANE DE BRUCH

(43) Date of publication of application: 16.04.2025
(73) Proprietor: Universität Münster, 48149 Münster (DE)
(72) Inventor: RUTSCH, Frank, 48149 Münster (DE); LANGER, Klaus, 48149 Münster (DE); NITSCHKE, Yvonne, 48149 Münster (DE)
(74) Representative: Schiweck Weinzierl Koch Patentanwälte Partnerschaft mbB

(56) References cited:
- US-A1- 2022 089 706
- US-B2- 10 688 061
- KEUTH JACQUELINE ET AL: "Reversion of arterial calcification by elastin-targeted DTPA-HSA nanoparticles", EUROPEAN JOURNAL OF PHARMACEUTICS AND BIOPHARMACEUTICS, ELSEVIER SCIENCE PUBLISHERS B.V., AMSTERDAM, NL, vol. 150, 6 March 2020 (2020-03-06), pages 108 - 119, XP086129927, ISSN: 0939-6411, [retrieved on 20200306], DOI: 10.1016/J.EJPB.2020.03.007
- HYUNCHEOL KIM ET AL: "Investigating the Movement of Intravitreal Human Serum Albumin Nanoparticles in the Vitreous and Retina", PHARMACEUTICAL RESEARCH, KLUWER ACADEMIC PUBLISHERS-PLENUM PUBLISHERS, NL, vol. 26, no. 2, 29 October 2008 (2008-10-29), pages 329 - 337, XP019686087, ISSN: 1573-904X
- RICHARD B THOMPSON ET AL: "Identification of hydroxyapatite spherules provides new insight into subretinal pigment epithelial deposit formation in the aging eye", PROC NATL ACAD SCI PROC NATL ACAD SCI, 3 February 2015 (2015-02-03), pages 1565 - 1570, XP055252778, Retrieved from the Internet <URL:http://www.pnas.org/content/112/5/1565.full.pdf> [retrieved on 20160224], DOI: 10.1073/pnas.1413347112

## Description

### TECHNICAL FIELD OF THE INVENTION

The present invention provides a nanoparticle comprising a scaffold comprising a biodegradable material, an antibody, that is able to target a component of a Bruch's membrane, and an anti-calcifying agent for use in a prophylaxis or in a treatment of a pathologic change of Bruch's membrane and/or an adjacent tissue, including a retinal pigment epithelium and a choroid of an eye. Additionally, the present invention provides a pharmaceutical composition comprising said nanoparticle and pharmaceutical acceptable excipients for use in a prophylaxis or in a treatment of a pathologic change of Bruch's membrane and/or adjacent tissues, including a retinal pigment epithelium and a choroid of an eye. Preferably said pathological change is a calcification of Bruch's membrane and/or the adjacent tissue, including the retinal pigment epithelium and the choroid of the eye.

### BACKGROUND

A Bruch's membrane (BM) is a pentalaminar structure located between a retinal pigment epithelium (RPE) and fenestrated choroidal capillaries of the eye. The Bruch's membrane is an elastin- and collagen- rich matrix that acts as a molecular sieve: it partly regulates an exchange of biomolecules, nutrients, oxygen, fluids and metabolic waste products between a retina and a systemic circulation (Booij *et al.,* 2010). The Bruch's membrane consists of five layers, namely, retinal pigment epithelium basal lamina, (RPE-BL), inner collagenous layer (ICL), elastic layer (EL), outer collagenous layer (ICL), and choriocapillaris basal lamina (Chc-BL). The elastic layer predominantly contains elastin in addition to fibronectin and other proteins and collagen VI (Booij *et al*., 2010). The functions of Bruch's membrane include 1) the regulation of the diffusion of (bio-) molecules between the choroid and RPE, 2) providing physical support for RPE cell adhesion, migration and perhaps differentiation, 3) acting as a division barrier, restricting choroidal and retinal cell migration (Booij *et al.,* 2010).

The Bruch's membrane and a subretinal space could be calcified as a symptom of various eye diseases. It has been found that angioid streaks found in eye diseases are associated with the calcification of Bruch's membrane. Angioid streaks are visible irregular crack-like dehiscences in Bruch's membrane, which are associated with atrophic degeneration of the overlying retinal pigmented epithelium (RPE) (Georgalas *et al.,* 2009). In angioid streaks, the elastic lamina that occupies the midsegment of BM is affected by calcification associated with disintegration of elastic fibers (Georgalas *et al*., 2009). Ultimately, the breaks in BM lead to neovascularization in the affected patients and loss in visual acuity (Hu *et al.,* 2003). Angioid streaks may be idiopathic in up to 50% of cases, but also occur in Pseudoxanthoma elasticum (in nearly all patients after 20 year of first diagnosis), Marfan's syndrome, Paget's disease of bone (10% of cases), sickle cell disease (in 1 - 2% of cases) and in other hemoglobinopathies, including beta thalassemia, diabetes mellitus and several others (reviewed by Tripathy *et al.,* 2021). Calcification of the elastic layer of BM is interpreted to be the major factor leading to its brittleness (Jampo *et al.,* 1987).

The calcification of Bruch's membrane is also associated with age and age-related macular degeneration (AMD). In a study in 1992 of 182 human maculae older than 33 years of age, a calcium deposition was found in BM in 59% of the samples. The presence of BM calcification was positively correlated with age, but not with age related macular degeneration (AMD) in this study (van der Schaft *et al.,* 1992). However, in 1999 a further study demonstrated a significant correlation between BM calcification and AMD. In this study, the authors concluded that "calcification of BM is an important factor that increases the brittleness of BM and enhances the chances of fibrovascular ingrowth" (Spraul *et al.,* 1999). In a study in 2015, five of six AMD donors, but only one of the seven controls showed nanocrystalline hydroxyapatite (HAP) calcifications at the level of the BM. The authors of this study were able to show that these calcifications were hydroxyapatite based, located in the elastic layer of BM and showed a "cage-like" structure, which "would facilitate additional storage of iron and lead in the calcified areas". The authors proposed that the calcification acts as a barrier, decreasing the permeability of BM. They concluded that this together with the accumulating heavy metal iron can facilitate degeneration of the overlying RPE and retina (Biesemeier *et al*., 2015). Independently, Thompson *et al.* identified hydroxyapatite spherules with cholesterol containing cores in subretinal deposits with proteins bound to these structures. They observed HAP spherule formation on the surface of lipid droplets in the inner aspect of Bruch's membrane and concluded that HAP can be directly formed in physiological concentrations of calcium and phosphate at physiological pH and that this process may occur in the sub-RPE space. The authors proposed a novel mechanism for the growth and the initiation of sub-RPE deposits with implications for the development of AMD: "Sub-RPE deposit-growth, and maybe even formation, may be mediated by the formation of HAP shells on cholesterol-containing, naturally present extracellular lipid droplets at the RPE-choroid interface." (Thompson *et al.,* 2015). Apart from the "spherules", which range from 0.5 to 10 µm in diameter, with the most frequent size of the distribution around 1.5 µm (Szmacinski *et al.,* 2020), a study by Tan *et al.* provided evidence that a subset of HAP deposits, which are larger (tens of micrometers in size) than the spherules and termed "nodules" were associated with increased risk (odds ratio 6.4) of progression to advanced AMD within one year, providing good evidence for a direct link between HAP deposition and advanced AMD (Tan *et al.,* 2018).

There is evidence that calcifications of Bruch's membrane are causally related to membrane breaks leading to angioid streaks, which can be associated with loss of visual acuity (Hu *et al.,* 2003). Furthermore, the study of Biesemeier proposed an additional mechanism, namely the "cage model" of hydroxyapatite, which on the one hand decreases the permeability of the BM and on the other hand leads to accumulation of further substances, which facilitate the degeneration of the overlying RPE and retina in the context of AMD (Biesemeier *et al.,* 2015). Additionally, hydroxyapatite containing "shells" containing lipid droplets in the RPE/choroid interface may mediate the growth and maybe even the formation of subretinal deposits, which in the end may progress to AMD (Thompson *et al.,* 2015). Finally, it was shown, that larger HAP containing nodules located in retinal drusen provided a direct link between HAP in subretinal deposits and disease progression in AMD (Tan *et al.,* 2018).

Regarding possible methods to treat Bruch's membrane calcification Booij *et al.* in 2010 stated that "further elucidation of the calcification process (in BM) holds the promise that soft tissue and BM calcification can perhaps be influenced by drugs or by dietary means" (Booij *et al.,* 2010). Indeed, in this respect, LaRusso *et al.* were able to show that elevated dietary magnesium prevented connective tissue mineralization in a mouse model of PXE. In this paper, the authors however focused on the mineralization of the connective tissue capsule surrounding the vibrissae and not on BM calcifications (LaRusso *et al*., 2009). Ethylenediamine-tetraacetic acid (EDTA) chelation therapy has been used in ophthalmology to treat calcific band keratopathy (CBK), a chronic degenerative condition characterized by the deposition of garish to whitish opacities in the superficial layer of the cornea, most frequently in the interpalpebral zone. CBK is referred to as primary or idiopathic. In the early stages, it remains asymptomatic; however, once it extends into the visual axis, it results in significant glare and visual disturbances. Various modalities have been used in the treatment of CBK, by far the most widely used method is EDTA chelation with the goal of removing calcium opacities and to restore a smooth ocular surface. Other methods include the use of a diamond burr, Nd:YAG laser, lamellar keratoplasty and phototherapeutic keratectomy. EDTA chelation therapy for CBK is performed at the slip lamp by preparing a 3.75% dilution of disodium EDTA in a tuberculin syringe. After the instillation of topical anesthesia, the epithelium overlying the band keratopathy is removed and a cellulose sponge or sterile cotton applicator is soaked in the diluted EDTA solution and rubbed against the calcium until its dissolution (Najjar *et al.,* 2004).

Keuth *et al.,* 2020 discloses a nanoparticle comprising a human serum albumin scaffold, a diethylenetriaminepentaacetic acid (DTPA) molecule and an Elastin antibody which is used for reducing the calcification in an aortic ring model ex-vivo.

Kim *et al.,* 2009 discloses HSA-NP which are promising drug delivery carrier for the treatment of AMD.

Thus, a better form of delivering chelating agents to the calcified Bruch's membrane and the adjacent tissues is needed.

### SUMMARY OF INVENTION

This object is accomplished, inter alia, by a nanoparticle for use in a prophylaxis or a treatment of a calcification of Bruch's membrane and/or an adjacent tissue and a pharmaceutical composition of the independent claims.

The present invention provides in a first aspect a nanoparticle comprising a scaffold comprising a biodegradable material, an antibody, that is able to target a component of a Bruch's membrane, and an anti-calcifying agent for use in a prophylaxis or a treatment of a pathological change of Bruch's membrane and/or an adjacent tissue, including a retinal pigment epithelium and a choroid of an eye.

In one embodiment of the nanoparticle for use of the present invention, said pathological change is a calcification of Bruch's membrane and/or the adjacent tissue, including the retinal pigment epithelium and the choroid of the eye.

In one embodiment of the nanoparticle for use of the present invention, said biodegradable material is human serum albumin.

In one embodiment of the nanoparticle for use of the present invention, said antibody is an anti-elastin antibody or an anti-vitronectin antibody and said antibody is preferably covalently linked to the nanoparticle.

In one embodiment of the nanoparticle for use of the present invention, said anti-calcifying agent is a chelator, preferably diethylenetriaminepentaacetic acid (DTPA) or ethylenediaminetetraacetic acid (EDTA), inorganic pyrophosphate (PPi), or sodium thiosulfate.

In one embodiment of the nanoparticle for use of the present invention, said nanoparticle has a negative surface charge.

In one embodiment of the nanoparticle for use of the present invention, said nanoparticle comprises a scaffold comprising human serum albumin, an anti-elastin antibody preferably covalently linked to the nanoparticle, and DTPA preferably covalently linked to the nanoparticle.

In a second aspect, the invention provides a pharmaceutical composition comprising the above mentioned nanoparticle and pharmaceutical acceptable excipients for use in a prophylaxis or in a treatment of a pathological change of Bruch's membrane and/or an adjacent tissue, including a retinal pigment epithelium and a choroid of an eye.

In one embodiment of the pharmaceutical composition for use of the present invention, said pathological change is a calcification of Bruch's membrane and/or an adjacent tissue, including a retinal pigment epithelium and a choroid of an eye.

In one embodiment of the nanoparticle for use or of the pharmaceutical composition for use of the present invention, said calcification of Bruch's membrane and/or adjacent tissues, including the retinal pigment epithelium and the choroid of an eye is a symptom of the disease.

In one embodiment of the nanoparticle for use or of the pharmaceutical composition for use of the present invention, said the disease is selected from the group consisting of Pseudoxanthoma elasticum, Angioid Streaks, peau d'orange fundus, Beta-Thalassemia, Sickle Cell disease, Juvenile Paget's disease, Hyperphosphatemic familial tumoral calcinosis (HFTC), ENPP1 deficiency, Idiopathic Sclerochoroidal calcification, Choroidal calcification associated to chondrocalcinosis (pseudogout), Sclerochoroidal Calcification with optic nerve calcification in primary hyperparathyroidism or parathyroid adenoma, Genetic renal tubulopathies: like Bartter syndrome, and Gitelman syndrome, Aplasia cutis congenita and oculoectodermal syndrome, Cytomegalovirus Retinitis, Age-related macular degeneration, Refractile drusen (a feature of AMD), and Optic Nerve Head Drusen.

In one embodiment of the nanoparticle for use or of the pharmaceutical composition for use of the present invention, said nanoparticle or said pharmaceutical composition is administered intravenously, intravitreally, subconjunctivally, subtenonly, retrobulbarly, posteriorly, juxtasclerally, suprachoroideally, subretinally, or topically as eye drops.

### BRIEF DESCRIPTION OF FIGURES

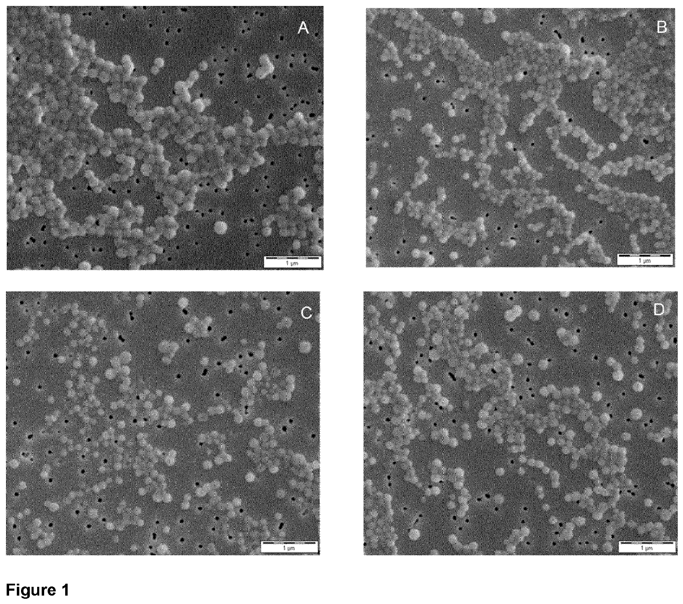

**Figure 1** Scanning electron microscopy images of HSA-NP (**A**), DTPA-(HSA-NP) (**B**), DTPA-(HSA-NP)-el ab (**C**), and DTPA-(HSA-NP)-IgG (**D**). The nanoparticles were prepared according to point 1.2.4 of the Materials and Methods section.

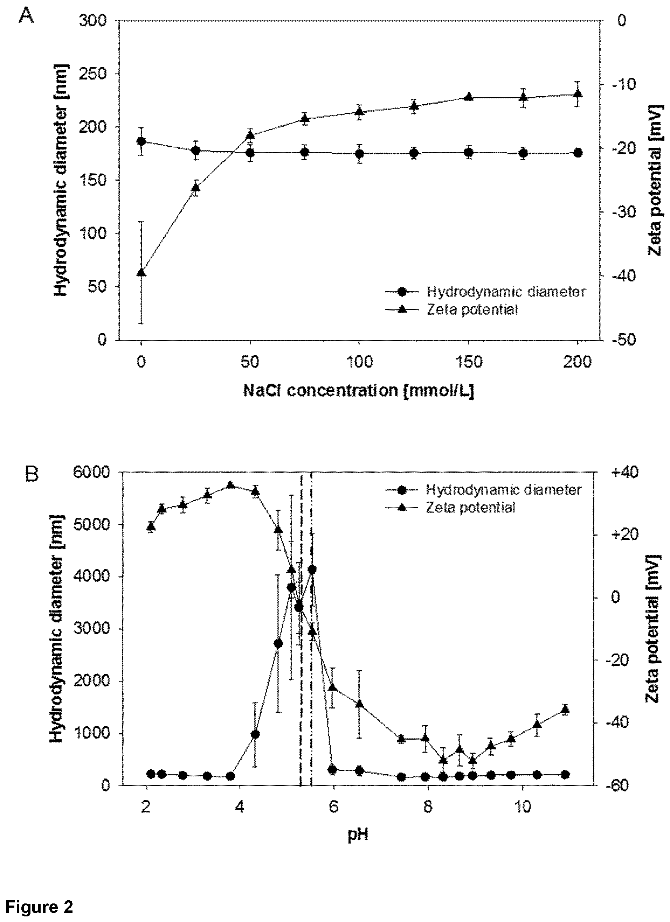

**Figure 2** The stability of DTPA-(HSA-NP) was evaluated over increasing electrolyte concentration (**A**) and a pH ranging from 2 to 11 (**B**) (mean ± SD, n = 3). Vertical lines in B represent the isoelectric points of HSA-NP (5.52 ± 0.07; -··-··-) and DTPA-(HSA-NP) (5.28 ± 0.11; - - -). The nanoparticles were prepared according to point 1.2.4 of the Materials and Methods section.
**Figure 3** Cell viability of (**A**) hCMEC/D3 cells, (**B**) Jurkat cells, and (**C**) primary macrophages and HUVECS after 24 h incubation with different NP formulations or pure DTPA (mean ± SD, n = 3). The nanoparticles were prepared according to point 1.2.4 of the Materials and Methods section.
**Figure 4** Cellular interaction of nanoparticles with hCMEC/D3 cells (**A**) and Jurkat cells (**B**) detected via live-cell imaging (mean ± SD, n = 3). The nanoparticles were prepared according to point 1.2.4 of the Materials and Methods section.
**Figure 5** Cellular interaction of nanoparticles with hCMEC/D3 cells (**A**) and Jurkat cells (**B**) measured via FACS analysis (mean ± SD, n = 3). The nanoparticles were prepared according to point 1.2.4 of the Materials and Methods section.
**Figure 6** Visualization of cellular interaction of different NP formulations with hCMEC/D3 cells after 24 h incubation. NP are visualized by red fluorescence of conjugated PF633P, cell nuclei were stained with DAPI (blue) and membranes with WGA Alexa Fluor^{®} 350 (blue). **A:** pHSA-NP; **B:** (pHSA-NP)-el ab; **C:** (pHSA-NP)-IgG; **D:** DTPA-(pHSA-NP); **E:** DTPA-(pHSA-NP)-el ab; **F:** DTPA-(pHSA-NP)-IgG. The nanoparticles were prepared according to point 1.2.4 of the Materials and Methods section.

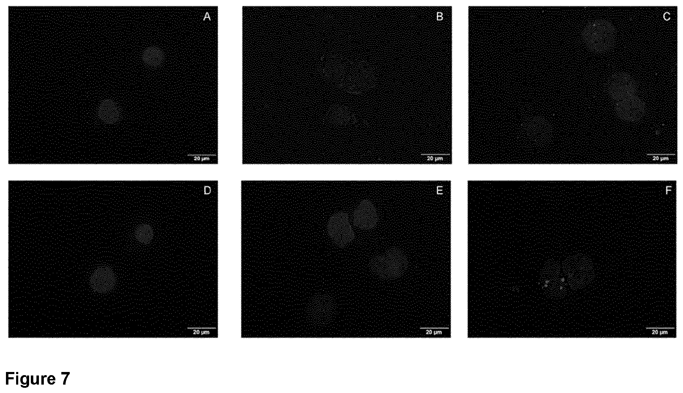

**Figure 7** Visualization of cellular interaction of different NP formulations with Jurkat cells after 24 h incubation. NP are visualized by red fluorescence of conjugated PF633P, cell nuclei were stained with DAPI (blue) and membranes with WGA Alexa Fluor^{®} 350 (blue). **A:** pHSA-NP; **B:** (pHSA-NP)-el ab; **C:** (pHSA-NP)-IgG; D: DTPA-(pHSA-NP); **E:** DTPA-(pHSA-NP)-el ab; **F:** DTPA-(pHSA-NP)-IgG. The nanoparticles were prepared according to point 1.2.4 of the Materials and Methods section.

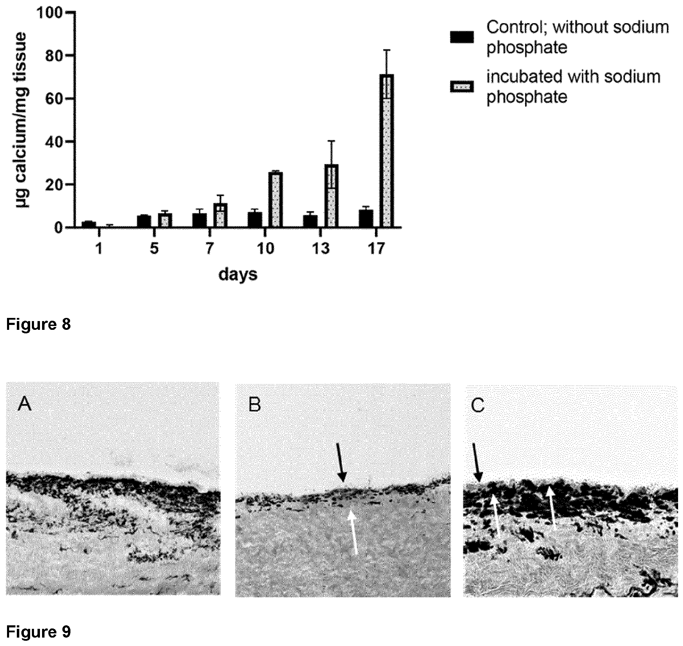

**Figure 8** Quantification of porcine ocular calcification after incubation in osteogenic medium at different time points. Degree of calcification is expressed in µg Ca^{2+/}mg tissue. Calcium content of tissue was quantified with the o-cresolphthalein method.
**Figure 9** Calcification of Bruch's membrane in porcine ocular tissue after 13 days in osteogenic medium. Calcified areas are stained in red. **A**) Ocular tissue in DMEM medium without sodium phosphate. **B** and **C.** Ocular tissue showing patchy Bruch's membrane calcifications (arrows). 2% Alizarin redS staining of ocular tissue including choroid and Bruch's membrane.

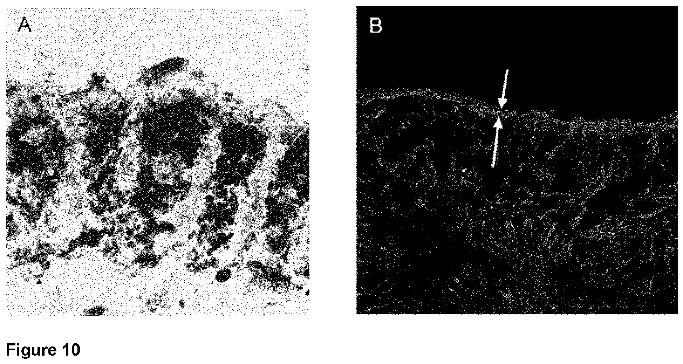

**Figure 10** Sustained vitality of choroid cells and strong elastin content in Bruch's membrane in porcine ocular tissue after 13 days in osteogenic medium. **A**) MTT staining of porcine ocular tissue after 13 days in osteogenic medium containing 5 mM NaH₂PO₄. **B**) Elastin immunofluorescence staining of porcine ocular tissue after 13 days in osteogenic medium containing 5 mM NaH₂PO₄ showing increased density of elastin in the region of Bruch's membrane (arrows).

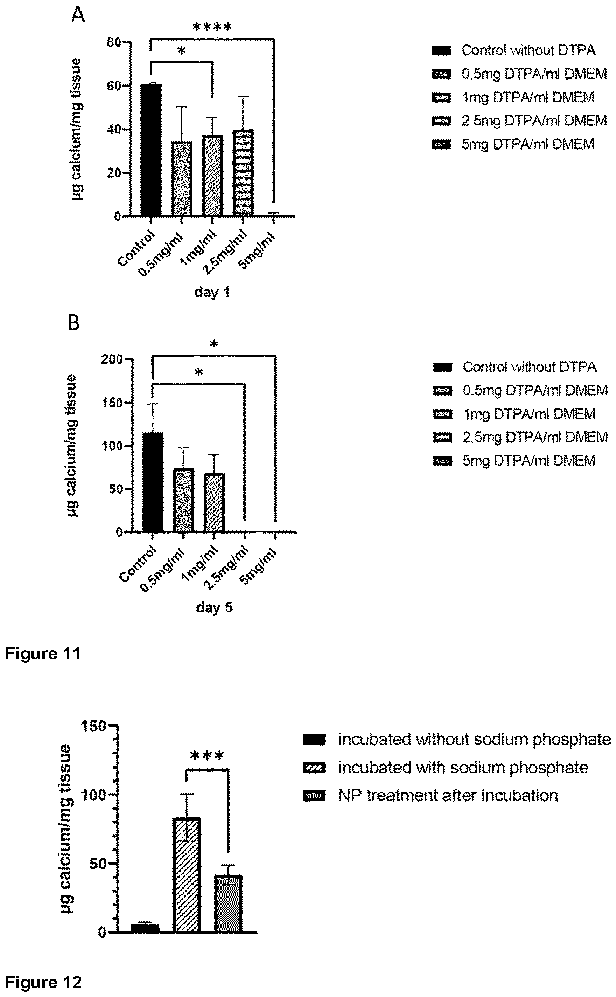

**Figure 11** Dissolution of Bruch's membrane calcifications by unbound DTPA. DTPA was added in different concentrations to porcine ocular tissue after 13 days in osteogenic medium. Calcium content of tissue was quantified with the o-cresolphthalein method one day after adding DTPA (**A**) and five days after adding DTPA to the tissue culture (**B**).*p<0.05; ****p<0.0001
**Figure 12** Dissolution of Bruch's membrane calcifications by DTPA coupled elastin targeted HSA nanoparticles (DTPA-(HSA-NP)-el ab). Nanoparticles were added to porcine ocular tissue after 13 days in osteogenic medium containing 5 mM NaH₂PO₄. Calcification content of tissue was quantified with the o-cresolphthalein method two days after addition of nanoparticles. Results represent 3 independent experiments. ***p<0.001. The nanoparticles were prepared according to point 1.2.4 of the Materials and Methods section.

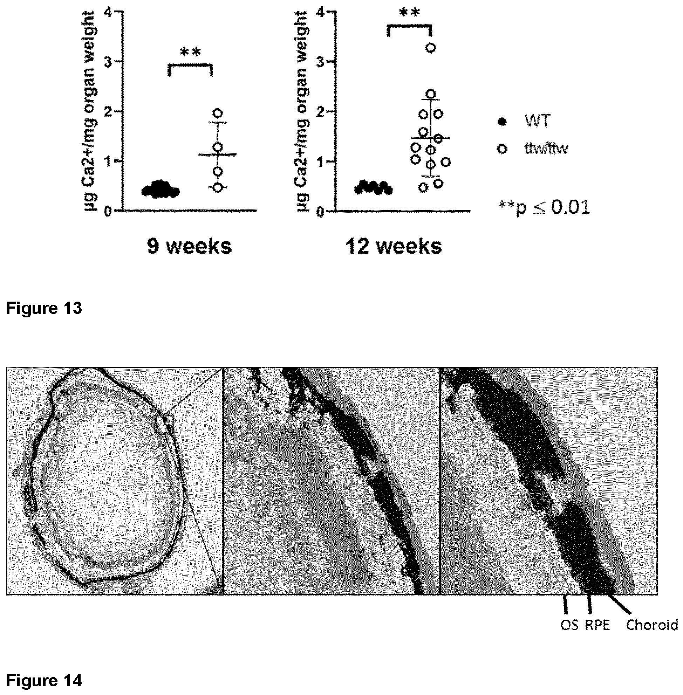

**Figure 13** Quantification of calcification of ocular tissue in ttw/ttw mice on a diet containing high phosphorus and low magnesium compared to wild-type mice on normal chow diet.
**Figure 14** Drusenoid calcification at the choroid- RPE interface in ttw/ttw mouse at 12 weeks of age. Middle panel and right panel show higher magnifications of insert of left panel. Alizarin Red S staining, cross section. OS: outer segment, RPE: retinal pigment epithelium. Choriod: Choriocapillaris

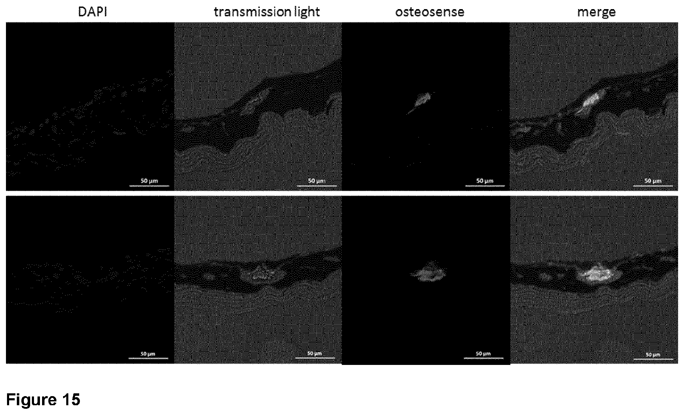

**Figure 15** Drusenoid calcification at the RPE-choroid interface in ttw/ttw mouse at 12 weeks of age (on acceleration diet). Cross section of flat mount sample, posterior segment of the eye, osteosense staining. Flat mounted sample was stained after careful removal of photoreceptor layer and of the RPE.

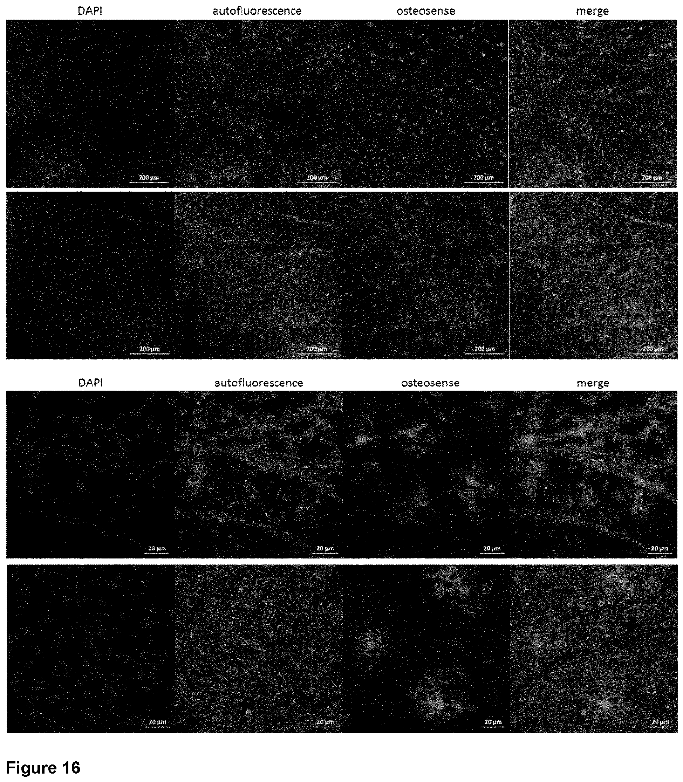

**Figure 16** Patchy calcifications at the level of the Bruch's membrane of the posterior segment of a ttw/ttw eye at 12 weeks of age (acceleration diet). Upper two panels: Snowflake pattern of calcifications. Lower two panels: patchy calcification on higher magnification. Flat mount section of the eye, stained after careful removal of the photoreceptor layer and of the RPE.

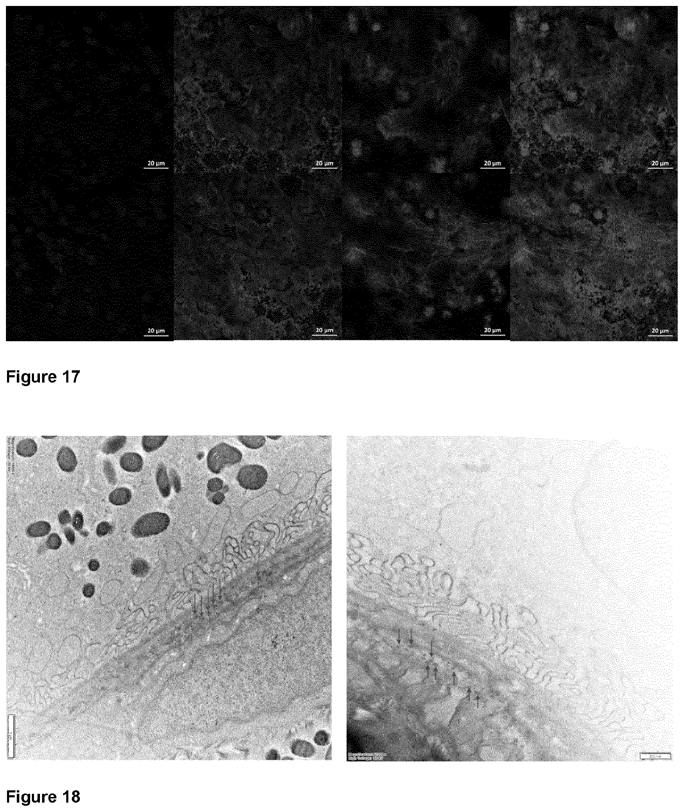

**Figure 17** Web-like calcifications at the level of the Bruch's membrane of the posterior segment of the eye of a ttw/ttw mouse at 12 weeks of age (acceleration diet). Flat mount sections of the eye were stained after careful removal of the RPE.
**Figure 18** Accumulation of magnetite-HSA-NP-el ab in Bruch's membrane of ttw/ttw mice. Magnetite-HSA-NP-el ab were injected in ttw/ttw mice i.v. After 4 hours, the mice were sacrificed and the eyes enucleated and prepared for transmission electron microscopy (TEM) imaging. TEM reveals an accumulation of the magnetite nanoparticles in Bruch's membrane (arrows). The nanoparticles were prepared according to point 1.2.6 of the Materials and Methods section.

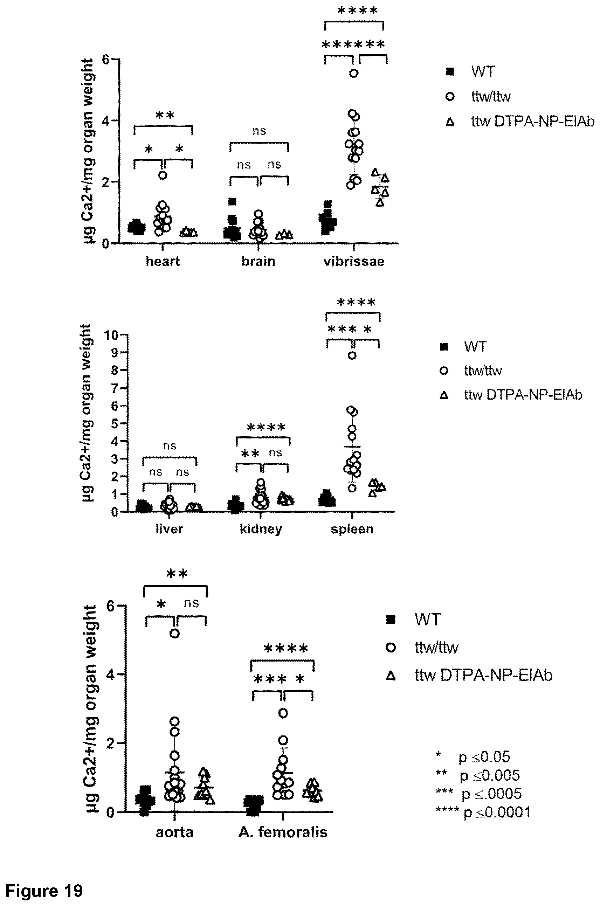

**Figure 19** Dissolution of calcifications in different organ tissues of ttw/ttw mice. DTPA bound HSA-based NP containing an anti-elastin antibody were injected into the tail vein of ttw/ttw mice on an acceleration diet. Injections started at the age of 10 weeks at a dose of 20 mg/kg and continued twice weekly for two weeks. Mice were sacrificed at the age of 12 weeks. Calcium content of organs from treated ttw/ttw mice was compared to untreated ttw/ttw mice and to wild-type mice of the same age. WT: wild type mice; ttw/ttw: Enpp1-deficient ttw/ttw mice on acceleration diet; ttw DTPA-NP-ElAb: ttw/ttw mice on acceleration diet treated with NP. The nanoparticles were prepared according to point 1.2.5 of the Materials and Methods section.

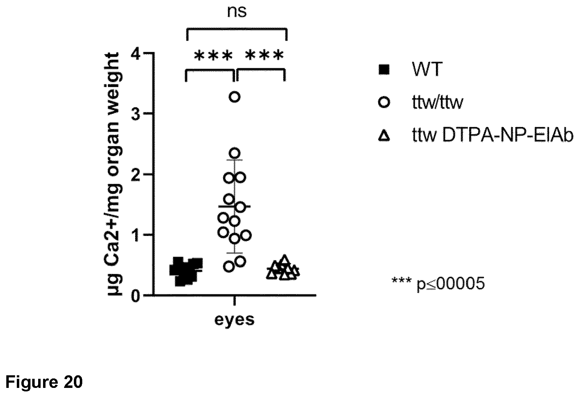

**Figure 20** Dissolution of ocular calcifications in ttw/ttw mice. DTPA bound HSA-based NP containing an anti-elastin antibody were injected into the tail vein of ttw/ttw mice on acceleration diet. Injections started at the age of 10 weeks at a dose of 20 mg/kg and were repeated twice weekly for two weeks (4 injections in total). Mice were sacrificed at the age of 12 weeks. Calcium content of eyes from treated ttw/ttw mice was compared to eyes from untreated ttw/ttw mice and to eyes from wild-type mice of the same age. WT: wild type mice; ttw/ttw: Enpp1-deficient ttw/ttw mice on acceleration diet; ttw DTPA-NP-ElAb: ttw/ttw mice on acceleration diet treated with NP. The nanoparticles were prepared according to point 1.2.5 of the Materials and Methods section.

### DETAILED DESCRIPTION OF THE INVENTION

A ttw/ttw mouse (tiptoe walking mouse) used herein as animal model has non-physiological calcifications, e.g. of an Achilles tendon, hair follicles of whiskers, and aorta (Hosoda *et al.,* 1981). It was shown that this ectopically calcification is due to a point mutation of a *NPPS* gene encoding a nucleotide pyrophosphatase (Npps), resulting in a non-functional truncated protein (Okawa *et al.,* 1998). This Npps is an ectonucleotide pyrophosphatase/phosphodiesterase 1 (ENPP1). Thus, the ttw/ttw mouse has an ENPP1 deficiency and further has cartilage calcification (Bertrand *et al.,* 2012). In humans, ENPP1 deficiency leads to generalized arterial calcification of infancy (Bäck *et al.,* 2019). In a further mouse model, the ENPP1 gene was knocked-out. This knock-out results in functional changes in the retina and calcification of Bruch's membrane resembling features of human diseases, like age-related macular degeneration (AMD), Pseudoxanthoma elasticum (PXE), and Generalized Arterial Calcification of Infancy (GACI) (Lengyel *et al.,* 2022). In an Abcc6 deficient mouse model of Pseudoxanthoma elasticum disease calcification of Bruch's membrane was detected (Gorgels *et al.,* 2012).

The inventors showed that ttw/ttw mice aged 9 to 12 weeks on an acceleration diet have calcifications of Bruch's membrane and adjacent tissues (shown in Example 7).

The inventors demonstrated that a nanoparticle comprising human serum albumin (HSA) as scaffold, an anti-elastin antibody, and magnetite accumulates in Bruch's membrane of ttw/ttw mice after intravenous injection (shown in Example 8 and Figure 18).

The inventors further showed that a nanoparticle comprising human serum albumin (HSA), an anti-elastin antibody, and a diethylenetriaminepentaacetic acid (DTPA) is able to resolve calcifications of Bruch's membrane in an *ex vivo* model using porcine eyes (shown in Example 6 and Figures 11 and 12).

Furthermore, the inventors succeeded in reducing the calcification of Bruch's membrane and adjacent tissue in ttw/ttw mice on an accelerated diet, using a nanoparticle comprising human serum albumin (HSA), an anti-elastin antibody, and a diethylenetriaminepentaacetic acid (DTPA) administered intravenously (shown in Example 9).

Thus, the inventors surprisingly found that a nanoparticle having a scaffold comprising a biodegradable material and further comprising an antibody, that is able to target the Bruch's membrane of an eye and an anti-calcifying agent is able to be used for the prophylaxis or treatment of a calcification of Bruch's membrane and/or an adjacent tissue, including a retinal pigment epithelium and a choroid of an eye.

The present invention provides therefore in a first aspect a nanoparticle comprising a scaffold comprising a biodegradable material, an antibody, that is able to target a component of a Bruch's membrane, and an anti-calcifying agent for use in a prophylaxis or a treatment of a pathological change of Bruch's membrane and/or an adjacent tissue, including a retinal pigment epithelium and a choroid of an eye.

It is preferred that the pathological change is a calcification of Bruch's membrane and/or the adjacent tissue, including the retinal pigment epithelium and the choroid of the eye.

This invention comprises the knowledge of pathological changes, e.g. a calcification of Bruch's membrane and/or adjacent tissue by e.g. calcium ions and the knowledge of the anatomical situation of Bruch's membrane and adjacent tissues. With the inventive use of the nanoparticle, the inventors at first use the possibility of an anti-calcifying agent suitable for the prophylaxis and/or treatment of the pathological change, e.g. an anti-calcifying agent that binds the ions, e.g. calcium ions, which are one causative for the calcification, and second to use a specific targeting, facilitated by an antibody specific for a component of Bruch's membrane and combining these uses with the nanoparticle comprising a biodegradable material. This results in a biocompatible nanoparticle, which is equipped with the antibody, for specifically binding to the Bruch's membrane, and the anti-calcifying agent. Since the nanoparticle is targeted to a component of Bruch's membrane this nanoparticle is predestinated for the use in the prophylaxis and treatment of the pathological change of Bruch's membrane and/or an adjacent tissue, e.g. the calcification of Bruch's membrane and/or an adjacent tissue.

The nanoparticle for the inventive use comprises the scaffold comprising the biodegradable material. Therefore, the nanoparticle could be degraded by enzymes of the human body without an unwanted deposit of a component of the material. Biodegradable materials usable for *in vivo* administration are known to the skilled person and could be found in pharmacopoeias or publications of a responsible pharmaceutical regulatory authority, like the FDA. Human serum albumin (HSA) is herein preferred as biodegradable material. HSA could be degraded in the human body and found to be safe by pharmaceutical regulatory authorities. Thus, HSA is ideal as biodegradable material used as the scaffold of the nanoparticle.

The nanoparticle for the inventive use further comprises the antibody, which is directed to the component of Bruch's membrane. Thus, the antibody, and therefore also the nanoparticle to which the antibody is bound, is specifically targeted to the component of Bruch's membrane. Since an antibody is able to specifically bind his cognate antigen, the antibody (and the nanoparticle bound thereto) is specifically directed to the component of Bruch's membrane, the antigen of the antibody. In general, any component of Bruch's membrane could be a suitable target or antigen of the antibody used herein. Preferably the targeted component is chosen from components mostly found in Bruch's membrane throughout the human body and in a suitable amount to circumvent binding of the antibody to its targeted component outside of Bruch's membrane. Suitable components may be elastin, vitronectin, fibronectin, or collagen VI. The preferred component for binding the antibody is elastin, which is the main component of the elastic layer of Bruch's membrane. The antibody is preferently covalently bound to the nanoparticle.

The term "antibody" as used herein and in the context of the present invention may comprise chimeric antibodies, humanized antibodies, monovalent antibodies, polyvalent antibodies, low-molecular antibodies, a diabody, a Fab fragment, or a scFv.

Chimeric antibodies refer to antibodies comprising variable and constant regions of different origins ligated with each other. For example, mouse-human heterogeneous chimeric antibodies are antibodies comprising the heavy and light chain variable regions of a mouse antibody and the heavy and light chain constant regions of a human antibody. Mouse antibody variable region-encoding DNAs are ligated with human antibody constant region-encoding DNAs, and the ligation products can be incorporated into expression vectors to prepare chimeric antibody-expressing recombinant vectors. Cells transformed with these vectors (recombinant cells) can be cultured for the expression of the DNA insert to obtain the chimeric antibodies produced during the culture.

In general, the chimeric antibodies comprise non-human animal-derived antibody variable regions and human antibody-derived constant regions. By contrast, the humanized antibodies comprise non-human animal-derived antibody complementarity-determining regions (CDRs), human antibody-derived framework regions (FRs), and human antibody-derived constant regions. The humanized antibodies are also called reshaped human antibodies. Specifically, for example, humanized antibodies comprising non-human animal (e.g., mouse) antibody CDRs grafted in human antibodies are known in the art. The humanized antibodies are useful as active ingredients for a therapeutic agent of the present invention, owing to their reduced antigenicity in the human body.

Each antibody variable region usually comprises 3 CDRs flanked by 4 FRs. The CDR regions substantially determine the binding specificity of the antibody. The CDRs have diverse amino acid sequences. On the other hand, amino acid sequences constituting the FRs often exhibit high homology among antibodies having different binding specificities. Therefore, in general, the binding specificity of a certain antibody can allegedly be transplanted to other antibodies through CDR grafting.

The antibody bound to the nanoparticle and present in a pharmaceutical composition of the present invention may encompass bivalent antibodies typified by IgG (IgG1, IgG2, IgG4, etc.) and also monovalent antibodies or polyvalent antibodies typified by IgM as long as these antibodies bind to the component of Bruch's membrane. The polyvalent antibody that may be present in the pharmaceutical composition of the present invention may encompass polyvalent antibodies having antigen-binding sites, all of which are the same as each other or some or all of which are different from each other.

The antibody bound to the nanoparticle and present in the pharmaceutical composition of the present invention may also be a low-molecular antibody that encompasses an antibody fragment deficient in a portion of the whole antibody (e.g., whole IgG). Such partial deficiency of the antibody molecule is accepted as long as the resultant antibody fragment is capable of binding to the component of Bruch's membrane. It is preferred that the antibody fragment employed in the pharmaceutical composition according to the present invention should contain one or both of heavy chain variable (VH) and light chain variable (VL) regions. It is also preferred that the antibody fragment employed in the pharmaceutical composition according to the present invention should contain CDRs. The number of CDRs contained in the antibody fragment employed in the pharmaceutical composition of the present invention is not particularly limited and is preferably at least 6 CDRs: heavy chain CDR1, CDR2, and CDR3 and light chain CDR1, CDR2, and CDR3.

The term "diabody" as used herein and in the context of the present invention may refer to a bivalent antibody fragment constructed by gene fusion. The diabody is a dimer comprising two polypeptide chains. Usually, each of the polypeptide chains constituting the dimer comprises heavy and light chain variable regions linked via a linker on the same chain. The linker in the diabody is generally too short to allow paring between heavy and light chain variable regions on the same chain. Specifically, the number of amino acid residues constituting the linker is, for example, approximately 5 residues. Therefore, heavy and light chain variable regions encoded on the same polypeptide chain cannot together form a single chain variable region fragment. Instead, they form a dimer by pairing with another single chain variable region fragment. As a result, the diabody has two antigen-binding sites.

The scFv, as used in the context of the present invention, may be obtained by linking heavy and light chain variable regions of the antibody. In the scFv, the heavy and light chain variable regions are linked via a linker, preferably, a peptide linker. The heavy and light chain variable regions in the scFv can be derived from any of the antibodies described in the present specification. The peptide linker that links the variable regions is not particularly limited. For example, an arbitrary single chain peptide of approximately 3 to 25 residues can be used as the linker.

The nanoparticle for the inventive use further comprises the anti-calcifying agent. The anti-calcifying agent could be any drug or agent that could be used for prophylaxis and/or treatment of the pathological change of Bruch's membrane and/or adjacent tissue. In the case that the pathological change of Bruch's membrane is the calcification of Bruch's membrane, the used agent is an anti-calcifying drug or anti-calcifying agent (both terms are used synonymously herein). This drug is needed for resolving and thereby treating the calcification of Bruch's membrane and/or the adjacent tissue. Ideally this drug could also bind the ions, e.g. calcium ions, responsible for the calcification such that the nanoparticle, to which the drug is bound, could also be used in the prophylaxis of the calcification. Such anti-calcifying drugs may be a chelator, which is able to bind divalent ions, like Ca²⁺. Suitable chelators are known to the skilled person. Preferably diethylenetriaminepentaacetic acid (DTPA) or ethylenediaminetetraacetic acid (EDTA) are used as chelator and anti-calcifying drug. Further preferred anti-calcifying drugs are inorganic pyrophosphate (PPi) and sodium thiosulfate. Especially preferred as anti-calcifying drug is diethylenetriaminepentaacetic acid (DTPA). The anti-calcifying drug could be covalently bound to the nanoparticle.

The nanoparticle used in the invention has preferably a negative surface charge. The surface charge could be expressed as zeta potential. The zeta potential is measured in that a part of the nanoparticle containing suspension is transferred in a special capillary cuvette. Using laser Doppler microelectrophoresis, the electrical mobility of the particles was measured on the Zetasizer Nano ZS (Malvern Instruments GmbH, Herrenberg, Germany) in zeta potential mode at an applied voltage of 140 V and a temperature of 22 °C. After evaluation with the Zetasizer device software, this velocity was transferred to the zeta potential output (e.g. described in Keuth *et al.,* 2020).

Thus, the especially preferred nanoparticle for use in the prophylaxis or treatment of the calcification of Bruch's membrane and/or the adjacent tissue, including retinal pigment epithelium and choroid of the eye comprises the scaffold comprising human serum albumin, the anti-elastin antibody preferably covalently linked to the nanoparticle, and DTPA preferably covalently linked to the nanoparticle. The preferred nanoparticle has a zeta potential of about -35.4 mV measured as described below and a hydrodynamic diameter of 179.8 nm. The hydrodynamic diameter is measured in that an aliquot of 10 µl of the purified particle suspension is diluted in a polystyrene cuvette with 2 ml ultrapure water to ensure free diffusion movement of the nanoparticles. The samples are measured with a Zetasizer Nano ZS (Malvern Instruments GmbH, Herrenberg, Germany) at a temperature of 22 °C and a backscattering angle of 173°. The mean hydrodynamic diameter and the intensity-weighted particle size distribution are calculated using the corresponding Zetasizer software (e.g. described in Keuth *et al*., 2020). Thus, the preferred nanoparticle is therefore suitable for intravitreal application due to its negative surface charge (cf. Kim *et al.,* 2009). Furthermore, the nanoparticle used in the *in-vivo* experiments has also the right size for taken up by Bruch's membrane after intravenous application. It was shown that nanoparticles with a diameter ranging from 270 nm to 420 nm could be delivered to laser treated eyes via intravenous injection resulting in that the healthy eye does not uptake the nanoparticle (cf. Singh *et al.,* 2009). Further, the preferred nanoparticle is specifically targeted to the component of Bruch's membrane due to the antibody coupled to the nanoparticle. This may result in lower side effects of the administered nanoparticle.

In a second aspect, the invention provides a pharmaceutical composition comprising the nanoparticle mentioned above and pharmaceutical acceptable excipients for use in a prophylaxis or in a treatment of a pathological change of Bruch's membrane and/or an adjacent tissue, including a retinal pigment epithelium and a choroid of an eye.

It is preferred that the pathological change is a calcification of Bruch's membrane and/or an adjacent tissue, including a retinal pigment epithelium and a choroid of an eye.

The above described nanoparticle for use and the pharmaceutical composition for use comprising the nanoparticle could be used for the prophylaxis and/or treatment of a disease, wherein the calcification, the pathological change of Bruch's membrane and/or adjacent tissues, including the retinal pigment epithelium and the choroid of an eye, is a symptom of the disease. As mentioned before the calcification of Bruch's membrane could be present or be a symptom of different diseases affecting the eye in general and especially disease affecting Bruch's membrane and/or adjacent tissues like the retinal pigment epithelium and the choroid.

In the following, examples of such diseases are mentioned together with their ICD 10 number or their orphan code (cf. https://www.orpha.net): Angioid streaks (ICD 10 number: H35.33), peau d'orange fundus, Pseudoxanthoma elasticum (Orpha code: 758), Beta-Thalassemia (Orpha code: 848), Sickle Cell disease (Orpha code: 232), Juvenile Paget's disease (Orpha code: 2801), Hyperphosphatemic familial tumoral calcinosis (HFTC) (Orpha code: 306661), and ENPP1 deficiency (Orpha code: 51608). Sclerochoroidal calcification is common in the following diseases Idiopathic Sclerochoroidal calcification, Choroidal calcification associated to chondrocalcinosis (pseudogout), (Orpha code: 1416), Sclerochoroidal calcification with optic nerve calcification in primary hyperparathyxroidism or parathyroid adenoma (Orpha Codes: 2207 and 417) (ICD 10: D35.1), Genetic renal tubulopathies, like Bartter syndrome (Orpha Code: 112) and Gitelman syndrome (Orpha Code: 358), and Aplasia cutis congenita and oculoectodermal syndrome Orpha codes 1114 and 3339). Retinal calcifications are a symptom of the following diseases: Cytomegalovirus Retinitis (ICD 10: B25.9), Age-related macular degeneration (ICD 10: H35.30; H35.31; H35.32), and Refractile drusen (a feature of AMD) (ICD 10: H35.36). Calcification of Bruch's membrane is also present in Optic Nerve Head Drusen (ICD 10: H47.32). In all the before mentioned diseases Bruch's membrane and/or the adjacent tissue is calcified. Therefore, the above described nanoparticle for use or pharmaceutical composition for use can be used for the prophylaxis and/or treatment of these diseases.

The inventive nanoparticle for use and the pharmaceutical composition for use could be administered via intravenous injection, intravitreal injection, subconjunctival injection, subtenon injection, retrobulbar injection, posterior juxtascleral injection, suprachoroideal or subretinal injection, or topical application as eye drops. Because of their negative surface charge, the nanoparticle is suited for intravitreal injection, since positive surface charged particles would not reach the posterior part of the eye (cf. Kim *et al.,* 2009).

It is to mention that therapeutic uses or methods mentioned herein are not forming part of the invention. However, the nanoparticle and the pharmaceutical composition as disclosed herein for use in a therapeutic method are part of the invention.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "and/or", wherever used herein, includes the meaning of "and", "or" and "all or any other combination of the elements connected by said term".

The term "less than" or in turn "more than" does not include the concrete number. For example, "less than 20" means less than the number indicated. Similarly, "more than" or "greater than" means more than or greater than the indicated number, e.g. "more than 80%" means more than or greater than the indicated number of 80%.

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step. When used herein the term "comprising" can be substituted with the term "containing" or "including" or sometimes, when used herein, with the term "having". When used herein, "consisting of" excludes any element, step, or ingredient not specified.

The term "including" means "including but not limited to", "Including" and "including but not limited to" are used interchangeably.

### EXAMPLES

### Material and Methods

### 1.1 Materials

Human serum albumin (HSA, purity > 96%), glutaraldehyde 50% (mA/) solution, and hydrocortisone were purchased from Sigma-Aldrich (Steinheim, Germany). Diethylenetriaminepentaacetic acid (DTPA) dianhydride was obtained from Thermo Fisher (Karlsruhe, Germany). The fluorescent dye PromoFluor-633P-NHS-ester (PF633P) was purchased from PromoCell GmbH (Heidelberg, Germany). The crosslinker NHS-PEG5000-Maleinimide (NHS-PEG₅₀₀₀-Mal) was purchased from JenKem Technology (Plano, USA). The anti-elastin antibody (polyclonal antibody to elastin -purified) was obtained from OriGene Technologies GmbH (Herford, Germany). IgG from rabbit serum was purchased from Sigma-Aldrich (Steinheim, Germany). Immortalized human T-lymphocyte cells (Jurkat) were purchased from Deutsche Sammlung von Mikroorganismen und Zellkulturen GmbH (Braunschweig, Germany) and the immortalized human cerebral microvascular endothelial cell line (hCMEC/D3) was purchased from Merck KGaA (Darmstadt, Germany). Roswell Park Memorial Institute (RPMI)1640, gentamicin, non-essential amino acids, L-alanyl-L-glutamine, sodium pyruvate, penicillin and streptomycin solution, trypsin/EDTA solution, phosphate buffered saline (PBS), and fetal bovine serum (FBS) were obtained from Biochrom AG (Berlin, Germany). Porcine hearts and porcine eyes were obtained from German Landrace pigs from a selected abattoir. ThermoFisher GmbH (Karlsruhe, Germany) provided o-cresolphthalein complexone grade as well as diethylenetriaminepentaacetic acid (DTPA). Antibodies used for immunofluorescence in porcine tissue culture are shown below:

| antibody | (animal) | name | Producer (location) | concentration |
|---|---|---|---|---|
| anti-elastin antibody | (mouse) | ab9519 | Abcam (Cambridge, USA) | 1:100 |
| anti-mouse IgG | (donkey) | AP192C | Sigma Aldrich (St. Louis, MO, USA) | 1:50 |

### 1.2 Nanoparticle preparation

### 1.2.1 Preparation of HSA nanoparticles

The albumin-based nanoparticles (HSA-NP) were prepared by using a previously described desolvation technique (Weber *et al.,* 2000). Briefly, the pH of an HSA solution (50 mg/ml, 1 ml) was adjusted to 7.3 with NaOH 2 N. The dropwise addition (1 ml/min) of 4 ml ethanol (96%, v/v) led to desolvation and thus formation of nanoparticles. The addition of 11.6 µL glutaraldehyde (8%, m/v) resulted in a theoretical crosslinking degree of 40%. After stirring with defined conditions (550 rpm, 22°C) overnight, the nanoparticles were purified by 2-fold centrifugation (21,000 × g, 16 min) and redispersed in a medium depending on further proceeding.

### 1.2.2 Preparation of PromoFluor 633P-labeled HSA-NP

HSA was labeled with the fluorescent dye PromoFluor-633P-NHS-ester (PF633P) prior to nanoparticle (NP) formation as described by Mesken *et al.* (Mesken *et al.,* 2017).1 mg PF633P was dissolved in 100 µL purified water. The solution was immediately added to 2 ml of an HSA solution (20 mg/ml) in bicarbonate buffer pH 8.3 and incubated for 1 h at 22°C, 600 rpm. Unbound PF633P was removed by purification using a dextran column (D-Salt^{™} Desalting Column, Thermo Scientific, Rockford, USA). After freeze drying of the labeled protein (pHSA), NP were prepared as described above by using a molar ratio of 1 pHSA to 5 unlabeled protein.

### 1.2.3 Covalent coupling of DTPA to the nanoparticle surface

A modified method of Hnatowich *et al.* (Michaelis *et al.,* 2004; Hnatowich *et al.,* 1983) was used to achieve a covalent binding of DTPA to HSA-NP. 9.08 mg DTPA dianhydride was added to 41.3 mg HSA-NP dispersed in NaHCO₃-buffer 0.05 M, pH 7.0 resulting in a molar excess of DTPA to HSA of 40:1. After stirring for 1 h (550 rpm, 22°C), unbound DTPA was removed by 3-fold centrifugation (20,000 × g, 14 min) and resuspension of the nanoparticles in purified water. After the final centrifugation, the DTPA bound HSA-NP (DTPA-(HSA-NP)), or if pHSA was used, DTPA-(pHSA-NP)) were redispersed in phosphate buffer pH 8.0 for antibody-conjugation.

### 1.2.4 Surface modification with antibodies (Synthesis route 1)

The conjugation of the antibody to the NP consisted of three steps. First, thiol groups were introduced onto the NP surface by a reaction of the HSA amine groups with 2-iminothiolane (Weber *et al.,* 2000a). A 50-fold molar excess of 2-iminothiolane over HSA was added to the NP dispersion in phosphate buffer pH 8.0. After incubation for 24 h at 22°C, 600 rpm in a thermomixer, the NP were purified twice by centrifugation (20,000 × g, 14 min) and redispersed in phosphate buffer pH 8.0. Meanwhile, either the anti-elastin antibody (el ab) or IgG were activated with the heterobifunctional crosslinker NHS-PEG₅₀₀₀-Mal. The crosslinker was used in 50-fold molar excess. Immediately after dissolving in phosphate buffer pH 8.0, NHS-PEG₅₀₀₀-Mal was added to 100 µg antibody followed by incubation for 1 h at 22 °C, 600 rpm. Subsequent, the thiolated NP were combined with the PEG-antibody complex and after incubation overnight (22°C, 600 rpm), the particles were purified by one-time centrifugation (18,000 g, 14 min) and redispersion in purified water. The resulting particles are hereinafter referred to as summarized in Table 1.

The surface modified NP according to synthesis route 1 were used for the experiments on NP characterization (Figure 1 and 2), cell interaction (Figure 3 - 7) and reversion of Bruch's membrane calcifications of porcine ocular tissue (Figure 12).

**Table 1 Abbreviations used for antibody-conjugated nanoparticles.**

| Nanoparticle system | Matrix material | Chelating drug | Conjugated antibody |
|---|---|---|---|
| (HSA-NP)-el ab | HSA | - | Elastin ab |
| (pHSA-NP)-el ab | pHSA | | |
| (HSA-NP)-IgG | HSA | - | IgG |
| (pHSA-NP)-IgG | pHSA | | |
| DTPA-(HSA-NP)-el ab | HSA | DTPA | Elastin ab |
| DTPA-(pHSA-NP)-el ab | pHSA | | |
| DTPA-(HSA-NP)-IgG | HSA | DTPA | IgG |
| DTPA-(pHSA-NP)-IgG | pHSA | | |

### 1.2.5 Surface modification with antibodies (Synthesis route 2)

The second antibody conjugation method used was characterized by the reversal of the crosslinking partners. In this case, the NP surface was first modified with the heterobifunctional crosslinker NHS-PEG5000-Mal. For this purpose, a 15-fold molar excess of the crosslinker in relation to HSA was added to the NP suspension. After incubation for 1 h at 22 °C, 600 rpm in a thermomixer, the NP were purified by centrifugation (13,000 × g, 13 min) and redispersion in phosphate buffer pH 8.0.

In parallel, the antibody was thiolated with a 50-fold molar excess of 2-iminothiolane in phosphate buffer pH 8.0 for 2 h at 600 rpm and 22 °C.

For covalent coupling, the thiolated antibody was incubated with the NP suspension (20 µg modified antibody / mg NP) overnight at 600 rpm and 22 °C. The resulting NP were purified by centrifugation (10,000 × g, 10 min) and redispersion in purified water. The surface modified NP according to synthesis route 2 were used for the experiments on reversion of calcification in ttw/ttw mice (Figures 19 and 20).

### 1.2.6 Preparation of magnetite-HSA nanoparticles

HSA was dissolved in ultrapure water at a concentration of 50 mg/ml. The pH was then adjusted to 8.5 using 2 M NaOH. Subsequently, 166.66 µl of a magnetite suspension (aqueous; 3% m/V) was added to 1 ml of the HSA solution. The pure mass of magnetite corresponded to 10% of the total mass of HSA. The magnetite-HSA solution was incubated for 1 h (20° C; 700 rpm). The further steps of particle preparation and functionalization followed analogously to the preparation of the surface modified NP according to synthesis route 2.

The magnetite-HSA nanoparticles were used for the accumulation experiments in Bruch's membrane of ttw/ttw mice (Figure 18).

### 1.3 Physicochemical characterization of nanoparticles

### 1.3.1 Photon correlation spectroscopy (PCS)

The average hydrodynamic diameter and the polydispersity index (Pdl) were determined by PCS measurement. After aqueous dilution, the NP suspensions were analyzed at a temperature of 22°C and a backscattering angle of 173°. The zeta potential - a dimension for the NP's surface charge - was measured by laser Doppler microelectrophoresis using the zeta potential mode. Both experiments were conducted using a Malvern Zetasizer Nano ZS system (Malvern Instruments Ltd., Malvern, UK).

### 1.3.2 Scanning electron microscopy images

Samples for the scanning electron microcopy images were prepared by applying 3 µl of a NP suspension (0.25 mg NP/ml) onto a polycarbonate membrane filter (pore width 0.1 µm). After drying in a desiccator overnight, the filter was sputtered with a thin gold layer. The images were taken with a CamScan CS4 scanning electron microscope (operating distance 10 mm, accelerating voltage 10 kV, Cambridge Scanning Company, Cambridge, UK).

### 1.3.3 Quantification of bound DTPA

The amount of bound DTPA was determined indirectly using a modified high-performance liquid chromatography (HPLC) method previously described by Yamaguchi *et al.* (Yamaguchi *et al.,* 1983). The supernatants resulting from purification after the coupling reaction were collected and a molar amount of iron(III) chloride equivalent to the maximum contained DTPA was added. Samples were analyzed using a HPLC-DAD system (Agilent Technologies 1200 series) combined with a reversed phase column (Gemini^{®} RP 18; 250 x 4.6 mm, particle diameter 5 µm (Phenomenex, Aschaffenburg, Germany), column temperature 30°C). A mobile phase consisting of phosphate buffer 0.02 M, pH 2.5, 0.175 mM tetrabutylammonium bromide and 5% acetonitrile was utilized for isocratic elution at a flow rate of 1.0 ml/min. Quantification was carried out using a calibration curve in a range of 0.1 - 1 mg DTPA/ml with a diode array detector (DAD) set to a wavelength of 257 nm for detection of the iron-DTPA-complex.

### 1.3.4 Verification of covalent binding of DTPA

The covalent binding of DTPA to the NP was confirmed by two methods.

First, the adsorption of DTPA to the NP surface was analyzed. Samples were prepared as described for the covalent DTPA coupling with the difference that DTPA dianhydride was not added to the NP suspension as dry powder but stirred in NaHCO₃-buffer 0.05 M, pH 7.0 for at least 1 h beforehand resulting in hydrolysis of the anhydride function. Afterwards, purification was performed as described above and the amount of DTPA adsorbed to the NP was determined using the specified HPLC method.

The second approach employed an *in vitro* release study to prove the persistence of the covalent linkage. NP dispersed in Dulbecco's Modified Eagle Medium (DMEM) at a concentration of 1 mg/ml were incubated using a thermomixer at 37°C, 600 rpm for defined time periods (30 min, 1 h, 2 h, 4 h, 6 h, 24 h). After the specified times, samples were centrifuged (20,000 × g, 15 min) to separate the NP. The supernatant was analyzed for free DTPA via HPLC-DAD as mentioned above.

### 1.3.5 Colloidal stability

The colloidal stability against the influence of electrolytes and pH was tested by performing titration experiments using the Malvern Zetasizer Nano ZS system in combination with a Multi-Purpose Titrator MPT-2 (Malvern Instruments Ltd., Malvern, UK). An aliquot of the NP suspension containing 5 mg NP was dispersed in purified water at a concentration of 0.38 mg/ml. For evaluating the stability against electrolytes, the titration was implemented using 0.6 M NaCl as titrant covering a range from 0 to 200 mM in 25 mM steps. The dispenser was set automatically and recirculation between measurements was activated for all measurements. For each electrolyte concentration the hydrodynamic diameter, Pdl, and zeta potential were measured. Titrations from pH 2 - 12 (increment: 0.5 pH intervals) were conducted using NaOH 0.05 N and HCl 0.05 N as titrants. The same parameters were determined as described for the electrolyte titration.

### 1.3.6 In vitro chelating ability

To evaluate the *in vitro* chelating ability of the prepared NP formulations, a method by Sarkar and Chauhan (*Sarkar et al*., 1967) was adapted. Briefly, an aliquot (1 ml) of a calcium chloride solution (10 µg/ml) was diluted to 1.375 ml with purified water, followed by the addition of 0.625 ml of an ammonium buffer pH 10.5 (0.24 g NH₄Cl in 100 ml NH₄OH 5%). 1 mg of NP was added and the volume filled up to 2.25 ml using purified water. After incubation for 5 min, NP were removed by centrifugation (20,000 × g, 15 min). An aliquot (0.25 ml) of o-cresolphthalein complexone (OCPC) solution 0.1% was added. Samples were analyzed using a Synergy MX multi-well spectrophotometer (BioTek Instruments GmbH, Bad Friedrichshall, Germany) set at a wavelength of 555 nm. A calibration curve of calcium ranging from 0 - 10 µg/ml calcium was prepared by following the steps described above but without NP addition.

### 1.3.7 Extent of antibody conjugation to the nanoparticles

The supernatants obtained through purification of the antibody-modified NP were analyzed with a GPC-VWD system to indirectly determine the extent of the antibody binding to the NP. An aliquot (20 µl) of the sample was injected onto a PSS Proteema column (PSS Proteema 300 A, 5 pm, 300 x 8 mm, Polymer Standards Service, Mainz, Germany) and eluted isocratically within 20 min at a flow rate of 1 ml/min, column temperature of 30°C. Phosphate buffer pH 6.6 was used as mobile phase. Detection wavelength was set at 280 nm. The column's separation capacity was tested by separation of the protein standard (Bio-Rad Laboratories GmbH, München, Germany) prior to each sequence.

The number of antibodies per one NP was calculated by first calculating the volume of one HSA-NP (V_{NP}) using the following equation (1): ${V}_{NP} = \frac{4}{3} π \times {\left(\frac{{d}_{h}}{2}\right)}^{3} ,$ (1) where dₕ represents the hydrodynamic diameter of HSA-NP.

Next, the mass of one HSA-NP (m_{NP}) was calculated using the equation (2) below: ${m}_{NP} = {V}_{NP} \times {ρ}_{NP} ,$ (2) where ρ_{NP} represents the density of HSA-NP which is 1.31 g/cm³ (Weber *et al.,* 2000a). By dividing the amount of the used NP for the antibody conjugation (m_{NP ab-conj.}) by m_{NP}, the total number of NP (Z_{NP}) was calculated (3): ${Z}_{NP} = \frac{{m}_{NP ab - conj .}}{{m}_{NP}} ,$ (3)

The number of antibody molecules (Z_{ab}) was calculated by dividing the mass of used antibody (m_{ab}) by the molar mass of the antibody (M_{ab}), followed by multiplication with the Avogadro constant (N_{A}) (4): ${Z}_{ab} = \frac{{m}_{ab}}{{M}_{ab}} \times {N}_{A} ,$ (4)

The number of antibody molecules per one NP (ab/NP) was calculated using the following equation (5): $\frac{ab}{NP} = \frac{{Z}_{ab}}{{Z}_{NP}} ,$ (5)

### 1.4. Cell culture experiments

### 1.4.1 Cell culture routines

hCMEC/D3 cells were cultivated using RPMI 1640 supplemented with 1% (v/v) non-essential amino acids, 1% (v/v) L-alanyl-L-glutamine (200 mM), 1% (v/v) gentamicin, 1% (v/v) sodium pyruvate (100 mM), 550 nM hydrocortisone, and 20% (v/v) FBS. Jurkat cells were cultivated using RPMI 1640 supplemented with 1% (v/v) of a mixture of penicillin (100 U/ml) and streptomycin (100 µg/ml), 1% (v/v) L-alanyl-L-glutamine (200 mM), 1% (v/v) gentamicin, and 10% (v/v) FBS. Both cell lines were stored in T75 cell culture flasks in an incubator at a temperature of 37°C and 100% humidity at 5% CO₂ atmosphere. The cells were split twice a week at a ratio of approximately 1:3. After thawing, all experiments were conducted within a maximum of 20 passages.

Human macrophages were isolated from buffy coats from peripheral blood of healthy volunteers obtained from the Red Cross by Ficoll gradient centrifugation subsequently followed by the isolation of CD14 positive cells by Magnetic Associated Cell Sorting (MACS, Miltenyi Biotec GmbH, Bergisch Gladbach, Germany). The purity of at least 95% of positive cells was determined by flow cytometry in an Agilent 2100 Bioanalyzer (Agilent Technologies Deutschland GmbH, Waldbronn, Germany). After isolation, cells were cultured in McCoy's 5A medium supplemented with 15% FBS, 1% L-glutamine, and 2% non-essential amino acids. The experiments were performed immediately after the isolation procedure at 37°C and 7% CO₂. Primary human umbilical vein endothelial cells (HUVECs) were obtained from PromoCell GmbH (Heidelberg, Germany). Briefly, the cells where cultured in Endothelial Cell Growth Medium 2 (ready-to-use) (PromoCell GmbH, Heidelberg, Germany) to confluency, subsequently followed by cytotoxicity experiments.

### 1.4.2 Cytotoxicity studies

To evaluate the cytotoxicity of the NP formulations a WST-1 assay (Roche Diagnostics, Mannheim, Germany) was conducted on Jurkat and hCMEC/D3 cell lines. Human macrophages and HUVECs were tested applying a XTT assay (Sigma-Aldrich Chemie GmbH, Munich, Germany). After seeding Jurkat and hCMEC/D3 cells in a 96 well plate at a density of 2 x 10⁴ cells/well (hCMEC/D3 cells suspended in 100 µL, Jurkat cells suspended in 50 µL), they were cultured for 48 h with serum containing medium. The NP influence on the cell viability was tested at concentrations ranging from 0.5 µg/ml to 250 µg/ml. Since Jurkat cells are suspension cells while hCMEC/D3 adhere to the well plate, the procedure for the two cell lines differed. Prior to the addition of the NP to hCMEC/D3 cells, the medium was removed. 100 µl of the NP suspension in serum free medium was added. After incubating for 24 h, the cells were washed once with serum free medium and 100 µl fresh serum free medium was added again. Following a blank measurement at 460 nm with a Synergy MX multi-well spectrophotometer, 10 µl of WST-1 reagent was pipetted into each well. After 30 min, the absorbance was measured again. To evaluate the viability, a negative and a positive control were conducted simultaneously to the nanoparticle incubation (100 µl of DMSO (0% viability) and 100 µL of serum free medium (100% viability)). The blank measurement was subtracted and the absorbances were correlated to the controls. For Jurkat cells, NP dilutions in serum free medium were twice as high as for hCMEC/D3 cells and 50 µl was added to the already present medium from seeding resulting in a 1:1 dilution to the final concentration. For the positive and negative control, 50 µl of DMSO and 50 µl of serum free medium was added, respectively. After 24 h of incubation, the blank measurement was performed without any washing steps. The remaining steps were equal to the ones described for the hCMEC/D3 cells.

The XTT assay was applied according to the manufacturer's protocol. Briefly, 2 x 10⁴ cells, suspended in 100 µl serum free medium, were seeded in a 96 well plate. In contrast to Jurkat and hCMEC/D3 cells, the viability of macrophages and HUVECs was investigated in the presence of only the highest concentration of 250 µg NP/ml. The cells were incubated for 24 h with the respective nanoparticles, followed by a washing step with serum free medium. After adding 100 µl fresh serum free medium, a blank measurement was conducted at 450 - 500 nm. XTT reagent was added in aliquots of 50 µl/well and incubated for 18 h at 37°C and 7% CO₂. The absorbance was measured and, after subtraction of the blank measurement, correlated to a negative control (cells in pure serum free medium).

### 1.4.3 Cellular interaction

To determine the cellular interaction of the NP formulations, three methods were employed. The first approach was conducted using an IncuCyte^{®} S3 Live-Cell Analysis Imaging System (Essen Bioscience, Inc., Michigan, USA). The second experiment was flow cytometry analysis. The obtained results were supported by visualization via fluorescence microscopy. All experiments were conducted using PF633P-labeled and therefore fluorescent NP.

### 1.4.3.1 Live-cell imaging

For analysis of NP interaction with cells, an IncuCyte^{®} S3 Live-Cell Analysis Imaging System was used. hCMEC/D3 cells were seeded at a density of 2 x 10⁵ cells/well in a 24 well plate. After cultivating for 48 h, the medium was exchanged with medium containing NP in a concentration of 0.02 mg/ml. Jurkat cells (50 µl) were seeded in an agarose-coated 96 well plate at a density of 1 x 10⁴ cells/well. An aliquot (50 µl) of a 0.04 mg/ml NP suspension was added resulting in a dilution to 0.02 mg NP/ml. The well plates were scanned every hour over a total time of 24 h whereby nine images per well were taken for the 24 well plate and 4 images for the 96 well plate at each time point. Fluorescent-labeled nanoparticles were detected by the image channel red (excitation: 565 - 605 nm / emission: 625 - 705 nm). The obtained red object areas of the samples minus the background of control wells containing medium without nanoparticles were set in relation.

### 1.4.3.2 Fluorescence-activated cell sorting (FACS)

To investigate the cellular interaction of the nanoparticles in more detail, FACS analysis was performed. hCMEC/D3 cells were seeded in a 24 well plate at a density of 2 x 10⁵ cells/well and cultivated for 48 h. 24 h before the measurement, the medium of three wells was exchanged with medium supplemented with 0.02 mg NP/ml for the first time point. For the remaining time points, the process was repeated 6 h, 4 h, 2 h, and 1 h prior measuring. After incubation, the medium was removed and cells were washed once with 300 µl trypsin/EDTA (0.05%/0.02%) solution. For enzymatic detachment of the cells another 300 µl trypsin/EDTA solution was added before placing the well plate in the incubator (37°C, 5% CO₂) for 7 min. 700 µL of medium was added to stop the enzymatic digestion and thereafter, samples were transferred into FACS tubes (Sarstedt, Nuembrecht, Germany) and analyzed using a FACSCalibur^{™} system and CellQuest^{™} Pro software, red laser 640 nm, filter 670LP, detector FL4 (Becton Dickinson Heidelberg, Germany). A minimum of 10,000 cells per sample was measured. The obtained mean fluorescence intensities were standardized to the fluorescence intensity of untreated control cells. For Jurkat cells, the experiment started directly after transferring 250 µL cell suspension (containing 1 x 10⁵ cells) in 2 ml micro tubes. The NP suspension was concentrated twice as high as required since 250 µl were added to the already present medium resulting in a 1:1 dilution to 0.02 mg NP/ml. The addition followed the same time protocol as described for hCMEC/D3 cells. Afterwards, the NP cell suspensions were directly transferred into FACS tubes. Analysis was carried out as described above.

### 1.4.3.3 Visualization of cellular interaction

For fluorescence microscopy, hCMEC/D3 cells were seeded with a density of 2.5 x 10⁴/chamber on Millicell^{®} EZ slides (Merck, Darmstadt, Germany). After a 24 h growing period, 0.625 µg NP (2.5 µg NP/ml) were incubated for 24 h. After washing the cells with PBS⁺⁺, they were incubated for 10 min with wheat germ agglutinin (WGA) Alexa Fluor^{®} 350 solution (10 µg/ml) to stain the cell membranes. Following another washing step with PBS⁺⁺, cells were fixed with paraformaldehyde 4% (m/v) for 15 min at room temperature. A final washing step was followed by covering the cells with Vectashield^{®} mounting medium with DAPI (Vector Laboratories Inc., Burlingame, USA). Since Jurkat cells are non-adherent cells, slides were coated with poly-L-lysine (PLL). Incubation with NP was carried out in 2 ml micro tubes. Therefore, 5 x 10⁴ cells were incubated with 1.25 µg NP (5 µg NP/ml) for 24 h. After centrifugation (1,000 × g, 5 min, 22°C), cells were resuspended in PBS⁺⁺. After incubation with wheat germ agglutinin (WGA) Alexa Fluor^{®} 350 solution (10 µg/ml) for 10 min, 20 µL of the cell suspension was pipetted onto the PLL-coated slides and left to adhere for 5 min. After a washing step with PBS⁺⁺, the remaining procedure for fixation and mounting was performed as described for hCMEC/D3. Samples were analyzed using an IX81 fluorescence microscope (Olympus, Hamburg, Germany) with filter systems including excitation at 360 - 370 nm, dichroic mirror at 400 nm, emission at 426 - 446 nm for DAPI and Alexa Fluor^{®} 350 and excitation at 575 - 630 nm, dichroic mirror at 636 nm, emission at 645 - 695 nm for PF633P. All pictures were taken as multi-layer image stacks with a minimum of 12 images. The stacks were processed by deconvolution ("Wiener filter") using cellSens Dimension Software version 1.8.1 (Olympus, Hamburg, Germany) to reduce out of focus fluorescence.

### 1.5 Ex vivo experiments

### 1.5.1 Preparation of the porcine eyes and tissue culture conditions

At first, the ocular muscles of enucleated porcine eyes were removed. The intact eyeball was put into 1x PBS (phosphate buffered saline) first, following a disinfection with Betaisodona (Mundipharma, Frankfurt am Main Germany) for five minutes. After rinsing the orange stain with the help of sterile 1x PBS, the subsequent steps followed under sterile conditions. A small incision was made with a lancet no. 22 at the corneoscleral junction serving as entry port for the surgical scissors cutting out the cornea circumferentially. In this process, the iris as well as the lens were removed. Through the resulting orifice the vitreous body was carefully dislodged. Proceeding sensitively, the neural retina was extracted easily as well. Next, with sharp surgical scissors, the ocular globe was cut into quarters. A trephine with a 10 mm diameter was used to punch out specimens of comparable sizes of the posterior part of the eye globe. Specimens were incubated in Dulbecco's Modified Eagle's Medium (DMEM) with 10% fetal bovine serum gold and penicillin/streptomycin at 37° Celsius and 5% CO₂. Furthermore, sodium phosphate (NaH₂PO₄/Na₂HPO₄) was added in a concentration of 5 mM to stimulate the calcification of ocular structures. The osteogenic medium was changed every second day.

### 1.5.2 Histology of ocular specimens

The porcine ocular specimen were incubated in conditioned medium for different amounts of time: at days 5, 7, 10, 13 and 17 after preparation they were washed in 1x PBS and were consequently embedded in Cryomatrix^{™} (Thermo Fisher Scientific, Schwerte, Germany) and immediately put into liquid nitrogen in order to accelerate the freezing process and to increase the chances of maintaining the structure of the different ocular layers. Object slides were produced with a chosen layer thickness of 20 µm. Alizarin Red S staining was performed to detect calcium compounds including hydroxyapatite using light microscopy (Paul *et al.,* 1983). Firstly, slides were washed in 1x PBS for five minutes, followed by a fixation within 95% ethanol for ten minutes. After letting slides thoroughly air dry, they were incubated in 2% aqueous Alizarin Red S for five minutes. The duration of this process depended on the mass of calcium in the chosen tissue. After rinsing the remaining Alizarin Red S in distilled water, the slides were dehydrated via standard procedure using graded alcohol follows as the final step, before mounting the slides with Roti Histokitt (Carl Roth GmbH + Co. KG, Karlsruhe, Germany). Samples were analyzed using light microscopy; photos were taken by Nikon DS-Ri2 and depicted via NIS-Elements Imaging Software (Nikon Instruments Inc., New York, USA).

### 1.5.3 Immunofluorescence of porcine ocular tissue

After thirteen days of pre-incubation, specimens were cryo-embedded and put on slides. After fixation in methanol for 15 minutes at -20°C, the samples were washed in 1x TBS (tris-buffered saline) and pre-treated with 2 mg/ml hyaluronidase in 0.5 M sodium acetate buffer with pH 6.0 at 37°C for 30 minutes. After blocking the sample for one hour by using 2% bovine serum albumin (BSA), the first monoclonal mouse antibody against elastin incubated 1:100 overnight. Following several washes in 1x TBS, the second antibody (donkey anti mouse IgG, 1:50) was incubated for one hour. After several washes in 1x TBS, slides were mounted in DakoCytomation fluorescent mounting medium (Dako, Hamburg, Germany). Images were taken using a Zeiss Axiovert 100 M laser-scanning microscope.

### 1.5.4 Calcium quantification in porcine ocular tissue

To minimize the chance of distortion by ectopic calcification, the choroid and the RPE were detached from the sclera and were treated isolated for the succeeding process. The resulting specimens underwent a drying process at 60°C for 48 hours, to then be weighed on a precision scale. Subsequently, a 24 hour incubation with 600 µl of 0.6 N HCl enabled the solution of phosphocalcific deposits. The amount of calcium was determined colorimetrically from the supernatants after centrifugation (15,000 rpm,10 minutes) using standard procedures of the o-cresolphthalein method (OCPC) (Saito *et al*., 2013). Calcification was expressed as µg of calcium per mg of tissue.

### 1.5.5 Visualization of cell survival in porcine ocular tissue

To ascertain the survival of the cells in the porcine ocular tissue in conditioned medium after thirteen days, the specimen were incubated for three hours in 0.5 mg/ml 3-(4,5-dimethylthiazol-2-yl)-2,5-diphenyltetrazoliumbromide (MTT) and subsequently washed in PBS, before they were cryoembedded and solidified by using liquid nitrogen. Due to metabolic activity, living cells induced the reduction of the yellow tetrazole into purple formazan.

### 1.5.6 Using DTPA as a chelating agent to resolve calcifications in porcine tissue

DTPA was the chosen calcium-chelating agent to dissolve calcifications. In order to identify the minimal effective amount necessary, DTPA was added after 13 days of pre-incubation in concentrations of 0.5 mg/ml, 1 mg/ml, 2.5 mg/ml and 5 mg/ml to the osteogenic medium. Afterwards, the specimens were taken out after one, three and five days to quantify the amount of residual calcification.

### 1.5.7 Nanoparticles used for dissolution of porcine ocular calcifications

Preparation of nanoparticles was done by Franziska Linß, Institute of Pharmacy, Muenster University, following a standard protocol. In brief, the applied agent consists of three parts: nanoparticles based on human serum albumin (HSA-NP), which hold a covalent binding of DTPA. As a last step, the conjugation to antibodies against porcine elastin led to the final product (DTPA-(HSA-NP)-AKEIastinPIG).

Characteristics of the used nanoparticle systems can be found below:

### Amount of bound DTPA per vial of nanoparticle system:

| Load | mg/vial | DTPA load (µg/mg HSA-NP) |
|---|---|---|
| 1 | 0.5 | 75.76 |
| 2 | 1 | 70.98 |
| 3 | 1 | 39.90 |

### 1.5.8 Using DTPA loaded nanoparticles to resolve Bruch's membrane calcifications in porcine ocular tissue

DTPA loaded nanoparticles were added after 13 days of pre-incubation at a concentration of 1.0 mg HSA bound DTPA/ml to the tissue culture in osteogenic medium. Afterwards, the specimens were taken out after two days to quantify the amount of residual calcification.

### 1.6 In vivo experiments

### 1.6.1 Animals and diet, dilution and application of NP, procedure of tail vein injection

The ttw/ttw mice used in this study have been described previously (Hosoda *et al.,* 1981; Okawa *et al.,* 1998). Ttw/ttw mice were bred onto a C57BL/6J background for more than ten generations, and ttw/ttw and wildtype (WT) littermate control (male and female) animals were generated through heterozygous mating. The study was approved by the local committee for animal studies (Reg. No.:81-02.04.2018.A233) and was performed according to the guidelines from Directive 2010/63/EU of the European Parliament on the protection of animals used for scientific purposes. Mice were genotyped after weaning and ttw/ttw mice were fed a high phosphate ("acceleration") diet (Altromin C1031 mod. #103153 with 0.4% calcium, 0.85% phosphorous and 0.04% magnesium) starting with 5 weeks of age.

Mice receiving nanoparticle treatment were injected i.v. twice a week, starting from 10 weeks of age (4 injections in total) into the lateral tail vein. For injection, DTPA-(HSA-NP)-ElastinAb were used. NP were injected in a maximum volume of 100 µl, in a concentration of 20 µg/g body weight, diluted in 0.9% NaCl. For NP injection, mice were weighed and placed in an animal restraint device. The veins of the mice were vasodilated prior to intravenous injection by prewarming of the tail in warm water (around 30°C) for 1-2 min, followed by disinfection of the tail. The needle was inserted into the distal portion of the middle 1/3 of the tail and the substance was injected. After injecting, the needle was left in tail for 2-3 seconds to allow the injected substance to clear the vein, then the needle was removed and immediately pressure was applied to the puncture site to prevent unnecessary blood loss. Once bleeding had stopped, the animal was removed from the restrainer. Mice were observed 5-10 min to ensure hemostasis (bleeding has stopped) and normal behavior of the animal.

All animals were sacrificed with 9 or 12 weeks of age by transcardial perfusion with 0.9% NaCl under deep anesthesia (5% isoflurane).

### 1.6.2 Histology of mouse eyes

### 1.6.2.1 Sectioning, preparation of samples and mounting

For histology, organs were fixed in 4% PFA for 24 hours, embedded in Cryomatrix^{™} (Thermo Fisher Scientific, Schwerte, Germany) and stored at -80°C until further processing. Cryo-embedded organs were cut into serial sections of 7 - 25 µm at the cryotome and transferred onto object slides. Slides were stored at -20 °C until further treatment.

For flatmount stainings of the eyes, eyes were enucleated and connective tissue was removed. Then the cornea, iris, lens and retina were gently removed. The eyecup was cut in each quarter to allow it to lie flat. The eye was incubated in 0.1% trypsin at room temperature for 15 min, followed by gentle brushing of the retinal pigment epithelium. Flatmounts were then fixed for 1 hour and stored at 4°C in PBS until further processing. For cross sections of the flatmount specimens, samples were uncovered from slides in PBS overnight followed by embedding in Cryomatrix^{™}. Cryo-embedded flatmounts were then cut into serial sections of 10 µm at the cryotome and transferred onto object slides. Slides were stored at -20 °C until further treatment.

### 1.6.2.2 Alizarin red staining

Slides were washed five minutes in PBS, fixed in 95% ethanol for 10 min at room temperature and then air dried. Samples were incubated five minutes with 2% alizarin red S pH 4.2, rinsed using aqua dest. and dehydrated (2 min in 70% EtOH, 5 min in 96% EtOH, 5 min in 99% EtOH, 5 min in 99% EtOH, 2 x 5 min in Roti^{®}Histol (Carl Roth, Karlsruhe, Germany)). Slides were covered with ROTI^{®}Histokitt (Carl Roth, Karlsruhe, Germany) and staining of red calcium mineral deposits was recorded using the Nikon eclipse ci microscope with Nikon NIS-Elements - Imaging Software (Nikon Europe BV Amsterdam, Netherlands).

### 1.6.2.3 Osteosense staining

Slides were washed in 1x PBS for five minutes. Then, slides were incubated with Osteosense 680EX (0.16 nmol/ml; NEV10020EX, Perkin Elmer, Waltham, US), a fluorescence labeled hydroxyapatite antibody, at room temperature for 20 minutes. Afterwards the slides were washed in PBS twice for five minutes followed by incubation with DAPI (1:1000 in PBS; 4',6-diamidino-2-phenylindole, D1306, Thermo Fisher Scientific, Schwerte, Germany) for nuclei staining for 15 minutes in the dark. Slides were washed three times for five minutes in PBS and covered with fluorescent mounting medium. Analysis of the staining was done using a Zeiss LSM 880 with Zen software (Carl Zeiss Microscopy GmbH, Jena, Germany).

Flatmounts were gently flattened on a slide and stained using the same protocol.

### 1.6.2.4 Transmission Electron Microscopy of mouse eyes

Eyes were isolated and immediately fixed in 2% (v/v) formaldehyde and 2.5% (v/v) glutaraldehyde in 100 mM cacodylate buffer, pH 7.4, at 4°C overnight. After washing in PBS, the eyes were post fixed in 0.5% (v/v) osmium tetroxide and 1% (w/v) potassium hexacyanoferrate (III) in 0.1 M cacodylate buffer for 2 h at 4°C followed by washing with distilled water. After dehydration in an ascending ethanol series from 30% to 100% ethanol, specimens were incubated two times in propylenoxide each for 15 min and embedded in Epon. Ultrathin sections were cut with an ultramicrotome, collected on copper grids, and negatively stained with 2% uranyl acetate for 10 min. Electron micrographs were taken at 60 kV with a Phillips EM-410 electron microscope using imaging plates (Ditabis, Pforzheim, Germany).

### 1.6.3 Calcification quantification

For calcium quantification, organs were dried at 60°C for two days. To solubilize phosphocalcific deposits, the organs were weighed and incubated in overnight 150 - 600 µl 0.6 N HCl, depending on the size of the organ. The organs were then sonicated for 10 seconds and centrifuged for 10 minutes with 13,000 rpm. 1% quinolinol solution (0.1 g quinolinol in 10 ml pure ethanol) and 0.1% o-cresolphthalein solution (0.01 g o-cresolphthalein in 7.2 ml aqua dest. and 2.8 ml ammonium chloride buffer [0.48 g NH₄Cl in 200 ml NH₄OH 5%]) were prepared. Various concentrations of CaCl₂*2H₂O in 0.6 N HCl were used as standard. 50 µl of each standard or sample were incubated with 110 µl ammonium chloride buffer and 20 µl of the 1% quinolinol solution for five minutes. At last, 20 µl of o-cresolphthalein were added and incubated for another five minutes. Calcium quantification was performed in duplicates. Calcium content was determined colorimetrically at a wavelength of 570 nm and normalized on dry weight.

### 1.7 Statistical methods

All experiments were performed in triplicate. The obtained results are presented as average value with standard deviation. To compare independent groups, a one-way ANOVA using the Holm-Sidak post-test was performed with the software SigmaPlot 12.5 (Systat Software GmbH Erkrath, Germany). For *ex vivo* experiments, statistical analyses were performed using Student's t-test (paired 2-sample testing for means). The significance levels are declared as * for p < 0.05, ** for p < 0.01, and*** for p < 0.001.

### Experimental examples

### Example 1 Preparation and characterization of nanoparticles

For the preparation of HSA nanoparticles according to point 1.2.4 of the Materials and Methods section, a desolvation method was utilized. HSA-NP with a crosslinking degree of 40% were obtained. These NP served as standard for the conducted experiments. The coupling reaction of DTPA dianhydride to the lysine residues of HSA lead to DTPA-(HSA-NP). The NP were conjugated to either the specific anti-elastin antibody or the unspecific IgG via a PEG-crosslinker. The results of the detailed physicochemical characterization of the prepared nanoparticle formulations, including the magnetite containing nanoparticles, are shown in Table 2 (below). The particle systems showed an average hydrodynamic diameter between 149 and 178 nm. A Pdl below 0.1 reflected a monodisperse size distribution for all samples. By means of SEM images, the PCS data were verified (Figure 1). Additionally, the spherical morphology of the nanoparticles was proven. HSA-NP exhibited a zeta potential of -22.6 ± 5.3 mV. The coupling of DTPA to the NP's surface led to a significant increase of the zeta potential of about 23 mV to -47.9 ± 7.0 mV (p ≤ 0.001). The antibody conjugated formulations were obtained with a zeta potential greater than -30 mV. Regarding the magnetite containing nanoparticles, the hydrodynamic diameter were 286 nm and 302 nm with a Pdl of 0.153 and 0.123. The zeta potential of the magnetite containing nanoparticles were -32.5 mV and -35.8 mV. The amount of bound DTPA was quantified by HPLC analysis of the collected supernatants during preparation (Table 2). PromoFluor-labeled formulations had a significant lower DTPA binding of 4.6 ± 0.1 mol DTPA/mol HSA (27.8 ± 0.6 µg DTPA/mg HSA) compared to 7.2 ± 1.1 mol DTPA/mol HSA (43.6 ± 6.7 µg DTPA/mg HSA) for the unlabeled formulations (p ≤ 0.05).

The covalent binding of DTPA to the nanoparticles was verified by two experiments. First, an adsorption study of DTPA to the NP's surface was conducted revealing that no DTPA was adsorbed to the NP surface. The whole DTPA amount used for the adsorption experiments was quantitatively recovered in the NP supernatant (data not shown). The second proof was given by a release study performed with the DTPA-modified NP. HPLC analysis of the supernatant after 24 h of nanoparticle incubation in serum free medium showed no DTPA peak in the chromatogram (data not shown). Consequently, no DTPA was released from the nanoparticles.

The extent of the NP surface functionalization with antibodies was evaluated. GPC analysis revealed that the antibody was quantitatively conjugated to the nanoparticle surface. Assuming an even distribution, 108 antibody molecules were bound per nanoparticle.

**Table 2 Physicochemical characteristics of prepared nanoparticles (mean ± SD, n ≥ 3).**

| Nanoparticle system | Hydrodynamic diameter [nm] | Pdl | Zeta potential [mV] | Drug load [mol DTPA/mol HSA] |
|---|---|---|---|---|
| HSA-NP/pHSA-NP | 158.1 ± 20.0 | 0.05 ± 0.03 | -22.6 ± 5.3 | -- |
| DTPA-(HSA-NP)/ DTPA-(pHSA-NP) | 166.9 ± 16.8 | 0.08 ± 0.06 | -47.9 ± 7.0 | 7.2 ± 1.1/4.6 ± 0.1 |
| (HSA-NP)-el ab/ (pHSA-NP)-el ab | 168.8 ± 22.1 | 0.10 ± 0.09 | -42.3 ± 10.1 | -- |
| (HSA-NP)-IgG/ (pHSA-NP)-IgG | 149.3^{a} | 0.04^{a} | -31.0^{a} | -- |
| Magnetite(10%)-HSA-el ab-NP | 302.5^{a} | 0.153^{a} | -35.8^{a} | -- |
| Magnetite(10%)-HSA-IgG-NP | 286.7^{a} | 0.123^{a} | ⁻32.5^{a} | -- |
| DTPA-(HSA-NP)-el ab/ DTPA-(pHSA-NP)-el ab | 155.3 ± 25.5^{b} | 0.04 ± 0.02^{b} | -49.0 ± 13.6^{b} | 7.2 ± 1.1/4.6 ± 0.1 |
| DTPA-(HSA-NP)-IgG/ DTPA-(pHSA-NP)-IgG | 178.4 ± 12.7^{b} | 0.08 ± 0.05^{b} | -40.5 ± 1.5^{b} | 7.2 ± 1.1/4.6 ± 0.1 |

| | | | | |
|---|---|---|---|---|
| Exception: ^{a} n = 1, ^{b} n = 2 | | | | |

### Example 2 Colloidal stability

To evaluate the particle systems' stability, electrolyte titrations and pH titrations were conducted. As seen in Figure 2A, the nanoparticles were stable over a sodium chloride concentration range up to 200 mmol/l. Figure 2B shows the pH titration ranging from pH 2 to 11. The isoelectric points (IEP), represented by vertical lines, of HSA-NP and DTPA-(HSA-NP) were detected at 5.52 ± 0.07 and 5.28 ± 0.11, respectively. Between pH 4.3 and 5.5, the hydrodynamic diameter of DTPA-(HSA-NP) increased reaching a maximum of 4,142 ± 696 nm which is significantly higher than the diameter below pH 4.3 or above pH 5.5 (p ≤ 0.001).

### Example 3 In vitro chelating ability

For a successful application in the dissolution of Bruch's membrane calcifications, DTPA-(HSA-NP) needed to exhibit chelating properties. Therefore, the chelating ability of the bound DTPA was evaluated. After incubating the NP in a calcium containing solution, they were removed by centrifugation and, after the addition of OCPC, the remaining calcium was quantified spectrophotometrically. As a result, 1 mg DTPA-(HSA-NP) chelated more than twice as much calcium compared to HSA-NP (2.69 ± 0.28 µg calcium/mg NP compared to 1.31 ± 0.13 µg calcium/mg NP) (**Table 3**).

**Table 3 Chelating ability of HSA-NP and DTPA-(HSA-NP) (mean ± SD, n = 3; *** for p ≤ 0.001).**

| Nanoparticle formulation | Chelated calcium [µg/mg NP] |
|---|---|
| HSA-NP | 1.31 ± 0.13 |
| DTPA-(HSA-NP) | 2.69 ± 0.28*** |

### Example 4 Cell culture experiments

The nanoparticles used in Example 4 were prepared according to point 1.2.4 of the Materials and Methods section.

### Example 4a Cytotoxicity

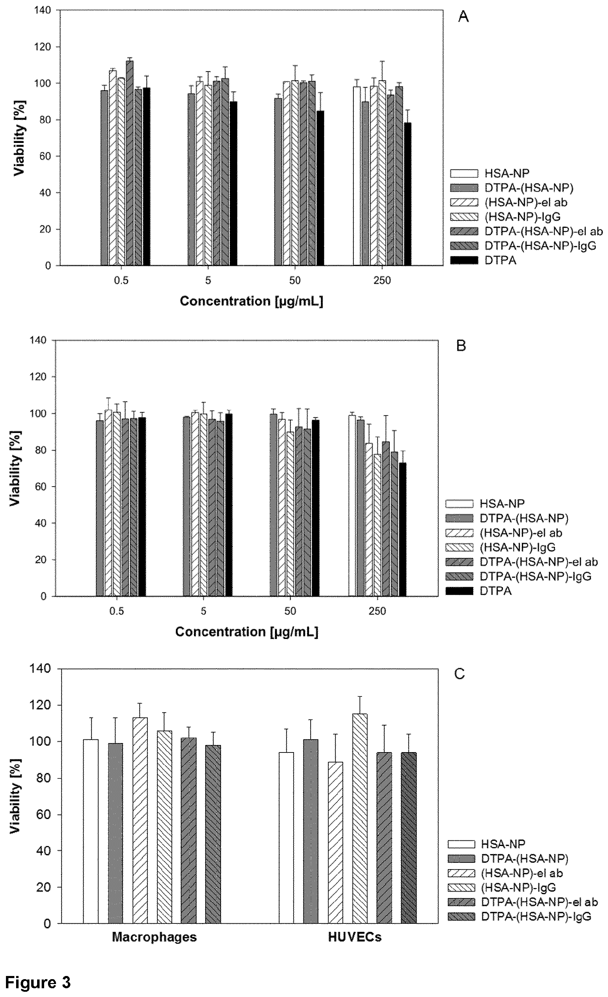

Since the NP were established to be applied into the human body, their biological safety is crucial. Thus, WST-1 and XTT assays were conducted to test the NP cytotoxicity. For Jurkat cells and hCMEC/D3, NP concentrations ranging from 0.5 to 250 µg/ml were tested. Pure DTPA was tested over the same concentration range. For primary macrophages and HUVECs, a NP concentration of 250 µg/ml was investigated. The results of the cytotoxicity studies are shown in Figure 3A and Figure 3B for hCMEC/D3 cells and Jurkat cells, respectively. The tested nanoparticulate formulations did not feature cytotoxic effects on hCMEC/D3 cells. Incubation with DTPA-(HSA-NP) resulted in the lowest viability with 89.9 ± 7.9% viable cells for 250 µg NP/ml related to incubation with pure medium. DTPA-(HSA-NP)-el ab and DTPA-(HSA-NP)-IgG impacted the cell viability comparable to the respective formulation without DTPA (around 98% cell viability). Pure DTPA lowered cell viability to 78.4 ± 6.9% at its highest concentration.

Jurkat cells exhibited a different sensitivity towards the formulations. Whereas DTPA-(HSA-NP) slightly reduced the number of viable cells to 96.5 ± 1.7%, the antibody-conjugated formulation exhibited a higher impact. (HSA-NP)-lgG obtained the lowest cell viability with 77.7 ± 9.6%. Nonetheless, the high standard deviations of the Jurkat cell viabilities for the highest NP concentration had to be respected resulting in no significance and therefore only a slight viability reduction. As observed for hCMEC/D3 cells, pure DTPA led to the highest decrease of cell viability to 73.0 ± 6.6%. For primary macrophages and HUVECs the cell viability was not influenced by the NP, maintaining values around 100% (Figure 3C).

### Example 4b Cellular interaction

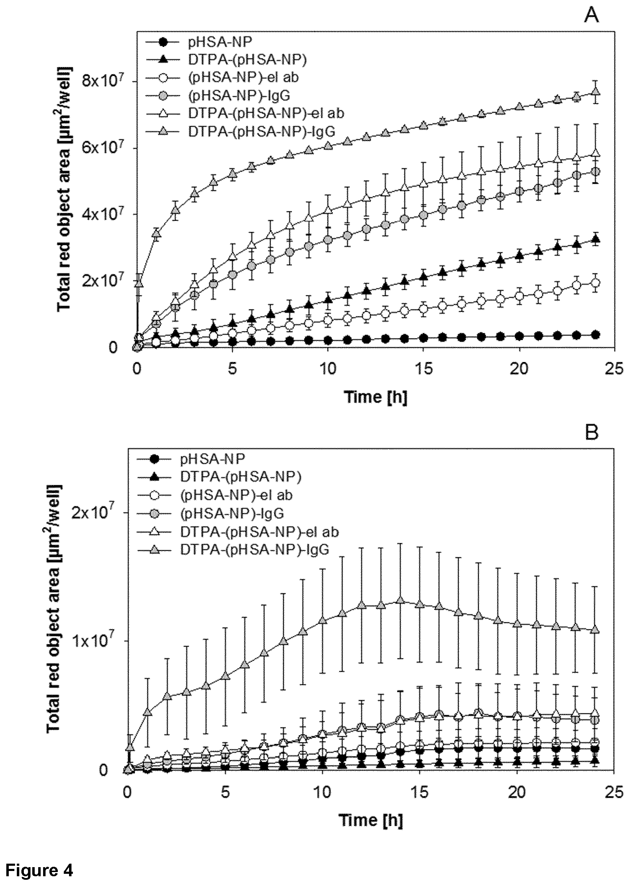

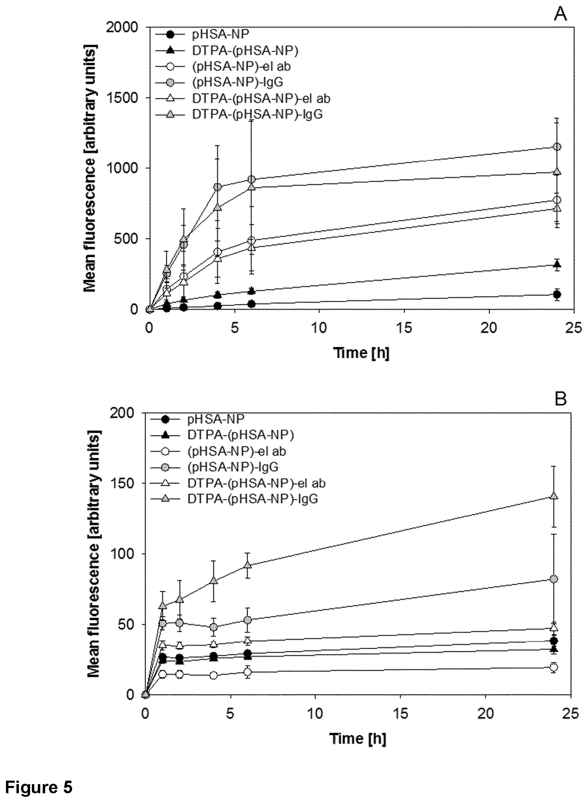

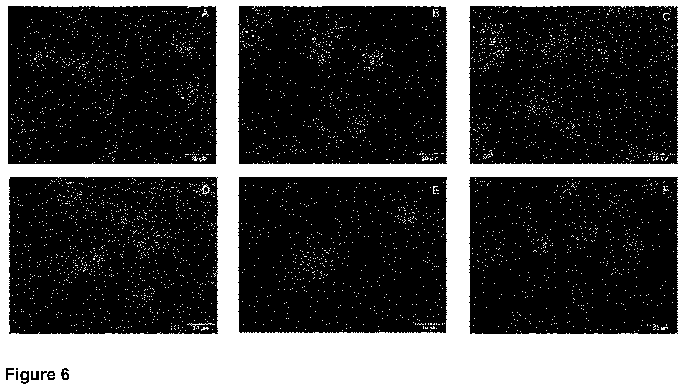

Precondition for the NP to reach the calcified elastic fibers is little to no interaction with endothelial cells or components of the blood. Live-cell imaging experiments showed a higher NP interaction with hCMEC/D3 cells (Figure 4A) compared to Jurkat cells (Figure 4B). However, the ranking of the interaction of the different NP formulations was comparable for both cell lines. DTPA-(pHSA-NP)-lgG showed the highest interaction followed by the el ab-bound formulations, while NP without conjugated antibody interacted less. Additionally, FACS analysis was performed to support the obtained live cell imaging data. Shear forces occurring during FACS analysis washed off only loosely attached NP from the cells leading to results more comparable to those under the influence of blood stream. The results obtained via IncuCyte^{®} were confirmed. hCMEC/D3 cells (Figure 5A) exhibited about ten times as much interaction as Jurkat cells (Figure 5B). Again, IgG conjugation led to the highest interaction followed by el ab-functionalized NP. NP missing a conjugated antibody interacted the least. For hCMEC/D3 cells, (pHSA-NP)-lgG and DTPA-(pHSA-NP)-lgG were significantly more associated to cells than HSA-NP and DTPA-(pHSA-NP), respectively, after 24 h (p ≤ 0.05). IgG-bound NP had interacted significantly more with Jurkat cells after 24 h as well (DTPA-(pHSA-NP)-IgG to DTPA-(pHSA-NP): p ≤ 0.001; (pHSA-NP)-lgG to pHSA-NP: p ≤ 0.01). For both cell lines conjugation of the anti-elastin antibody to DTPA-(pHSA-NP) resulted in significantly less cellular interaction than DTPA-(pHSA-NP)-lgG (p ≤ 0.001). Fluorescence microscopy visually confirmed the quantification data (Figures 6 and 7). Cells without nanoparticle incubation served as control and exhibited no red fluorescence (data not shown). pHSA-NP (Figure 6A) showed no interaction with hCMEC/D3 cells and only occasional red fluorescent NP could be detected. DTPA-(pHSA-NP) (Figure 6D) seemed to cover the cells in a haze rather than showing cell interaction. Antibody-conjugated nanoparticles ((pHSA-NP)-ab Figure 6B and C, DTPA-(pHSA-NP)-ab Figures 6E and F) showed more cellular interaction although it appeared to be more membrane associated rather than an uptake of the NP. For Jurkat cells (Figure 7) very little NP could be detected. Only IgG-conjugated nanoparticles (Figures 7C and F) were localized at the cells. Briefly summarized, all experiments to evaluate the cellular interaction revealed only little cell association.

### Example 5 Establishment of a porcine model of Bruch's membrane calcification

To establish a porcine model of Bruch's membrane calcifications, porcine ocular tissue cultures were prepared as described above and ocular tissue was cultured in conditioned medium containing 5 mM sodium phosphate. The inventors observed increasing calcium content of ocular tissue depending on the duration of days in culture in osteogenic medium (Figure 8). After 13 days in culture, reproducible positive Alizarin Red S staining of Bruch's membrane was visible, suggesting hydroxyapatite formation (Figure 9). Choroid cells were still viable at this time point as was demonstrated by positive MTT staining (Figure 10). Anti-elastin immunofluorescence staining confirmed a dense elastin content of Bruch's membrane (Figure 10).

### Example 6 Dissolution of Bruch's membrane calcifications in porcine ocular tissue

The inventors observed that calcium compounds of Bruch's membrane in porcine ocular tissue culture were resolved through addition of DTPA in increasing concentrations to the tissue culture in conditioned medium containing 5 mM NaH₂PO₄. The inventors demonstrated a reduction of tissue calcium content of one third compared to baseline in ocular tissue incubated with unbound 1 mg/ml DTPA and a 100% reduction of tissue calcium content in ocular tissue incubated with 5 mg/ml DTPA (Figure 11). Finally, porcine ocular tissue cultures, after 13 days in osteogenic medium were incubated with a nanoparticle solution containing 1 mg/ml DTPA bound to elastin targeted HSA based nanoparticles. Addition of the nanoparticle solution led to a 50% reduction of calcium content in ocular tissue, including Bruch's membrane (Figure 12).

### Example 7 Establishment and characterization of an in vivo mouse model displaying Bruch's membrane calcifications and drusen like calcifications

In 2022, Lengyel *et al.* reported Bruch's membrane (BM) calcification in ENPP1 deficient mice. ENPP1 transgenic mice were generated by breeding mice with loxP sites flanking ENPP1 exon 9 with mice expressing Cre recombinase behind a CAG synthetic germline promoter. BM calcification was observed in mice carrying the homozygous exon 9 deletion at 6 months of age (Lengyel *et al.,* 2022). To generate a mouse model with more robust and faster occurring BM calcifications the inventors used ttw/ttw mice. These mice are carrying a homozygous naturally occurring nonsense mutation in the *Enpp1* gene (Okawa *et al.,* 1998) and were subjected to a diet with a high phosphorus and low magnesium content starting at 5 weeks of age to accelerate the calcification process (Li et al., 2013). Ttw/ttw mice on this acceleration diet developed ocular calcifications at the age of 9 weeks. Ocular calcifications were even more profound at 12 weeks of age (Figure 13). Calcifications presented as drusenoid solid deposits at the interface between the choroid and the RPE (Figures 14 and 15) and as snow flake and web-like structures at the level of the Bruch's membrane (Figure 16 and 17).

### Example 8 Accumulation of magnetite-HSA-NP-el ab at the calcified Bruch's membrane of ttw/ttw mice

To show if nanoparticles comprising HSA and the elastin antibody could reach the calcified Bruch's membrane of ttw/ttw mice on acceleration diet (see above), the inventors prepared magnetite nanoparticles comprising HSA and the elastin antibody to enable electron microscopic imaging. The magnetite-HSA-NP-el ab were injected via the tail vein into ttw/ttw mice on acceleration diet. 4 hours after the intravenous injection the mice were sacrificed and the eye tissue prepared as described above. Using an electron microscope the magnetite-HSA-NP-el ab could be seen as black dots at the Bruch's membrane of ttw/ttw mice (Figure 18). Thus, magnetite-HSA-NP-el ab are able to accumulate at the calcified Bruch's membrane of ttw/ttw mice.

### Example 9 Dissolution of BM calcifications and drusen like calcifications in vivo

The nanoparticles used in Example 9 were prepared according to point 1.2.5 of the Materials and Methods section. To investigate the potential of DTPA bound HSA based nanoparticles coupled to anti-elastin antibodies to resolve existing calcifications *in vivo*, the inventors started to inject the NP systemically into the lateral tail vein of ttw/ttw mice on acceleration diet at 10 weeks of age at a dose of 20 mg/kg. At this time point, ectopic calcifications were already reliably present in the eyes of this animal model (Figure 13). Mice were injected intravenously (i.v.) twice a week with the same dose into the lateral tail vein (4 injections in total) until they were sacrificed at the age of 12 weeks. Systemic administration of the NP formulation led to a significant dissolution of ectopic calcifications of the heart (p ≤ 0.05), the sheaths of the vibrissae (p ≤ 0.0005), the spleen (p ≤ 0.05) and the femoral arteries (p ≤ 0.005) in ttw/ttw mice subjected to an acceleration diet (Figure 19). Systemic administration of the NP formulation also led to a dissolution of ocular calcification in this model, which was highly significant (p ≤ 0.0005) (Figure 20). The degree of ocular calcification after the administration of the NP formulation in the ttw/ttw mouse model did not differ from the degree of ocular calcification observed in wild-type mice of the same age (Figure 20).

### REFERENCES

Bertrand J, Nitschke Y, Fuerst M, Hermann S, Schäfers M, Sherwood J, Nalesso G, Ruether W, Rutsch F, Dell'Accio F, Pap T. Decreased levels of nucleotide pyrophosphatase phosphodiesterase 1 are associated with cartilage calcification in osteoarthritis and trigger osteoarthritic changes in mice. Ann Rheum Dis. 2012 Jul;71(7):1249-53.
Biesemeier A, Yoeruek E, Eibl O, Schraermeyer U. Iron accumulation in Bruch's membrane and melanosomes of donor eyes with age-related macular degeneration. Exp Eye Res. 2015 Aug;137:39-49.
Booij JC, Baas DC, Beisekeeva J, Gorgels TG, Bergen AA. The dynamic nature of Bruch's membrane. Prog Retin Eye Res. 2010 Jan;29(1):1-18.
Bäck M, Aranyi T, Cancela ML, Carracedo M, Conceição N, Leftheriotis G, Macrae V, Martin L, Nitschke Y, Pasch A, Quaglino D, Rutsch F, Shanahan C, Sorribas V, Szeri F, Valdivielso P, Vanakker O, Kempf H. Endogenous Calcification Inhibitors in the Prevention of Vascular Calcification: A Consensus Statement From the COST Action EuroSoftCalcNet. Front Cardiovasc Med. 2019 Jan 18;5:196.
Georgalas I, Papaconstantinou D, Koutsandrea C, Kalantzis G, Karagiannis D, Georgopoulos G, Ladas I. Angioid streaks, clinical course, complications, and current therapeutic management. Ther Clin Risk Manag. 2009 Feb;5(1):81-9.
Gorgels TG, Teeling P, Meeldijk JD, Nillesen ST, van der Wal AC, van Kuppevelt TH, Bergen AA. Abcc6 deficiency in the mouse leads to calcification of collagen fibers in Bruch's membrane. Exp Eye Res. 2012 Nov;104:59-64.
Hnatowich DJ, Layne WW, Childs RL, Lanteigne D, Davis MA, Griffin TW, Doherty PW. Radioactive labeling of antibody: a simple and efficient method. Science. 1983 May 6;220(4597):613-5.
Hosoda Y, Yoshimura Y, Higaki S. A new breed of mouse showing multiple osteochondral lesions--twy mouse. Ryumachi. 1981;21 Suppl:157-64.
Hu X, Plomp AS, van Soest S, Wijnholds J, de Jong PT, Bergen AA. Pseudoxanthoma elasticum: a clinical, histopathological, and molecular update. Surv Ophthalmol. 2003 Jul-Aug;48(4):424-38.
Jampol LM, Acheson R, Eagle RC Jr, Serjeant G, O'Grady R. Calcification of Bruch's membrane in angioid streaks with homozygous sickle cell disease. Arch Ophthalmol. 1987 Jan;105(1):93-8.
Kim H, Robinson SB, Csaky KG. Investigating the movement of intravitreal human serum albumin nanoparticles in the vitreous and retina. Pharm Res. 2009 Feb;26(2):329-37.
Keuth J, Nitschke Y, Mulac D, Riehemann K, Rutsch F, Langer K. Reversion of arterial calcification by elastin-targeted DTPA-HSA nanoparticles. Eur J Pharm Biopharm. 2020 May;150:108-119.
LaRusso J, Li Q, Jiang Q, Uitto J. Elevated dietary magnesium prevents connective tissue mineralization in a mouse model of pseudoxanthoma elasticum (Abcc6(-/-)). J Invest Dermatol. 2009 Jun;129(6):1388-94.
Lengyel I, Brown CN, Augustine J, Friedel T, Cunningham F, MacRae V, Kortvely E, Pilgrim M. Ectopic calcification in the Bruch's membrane and functional changes in the retina are associated with exon 9 deletion in ENPP1 in a transgenic mice model. Invest. Ophthalmol. Vis. Sci. 2022;63(7):464 - A0001 (abstract)
Li Q, Guo H, Chou DW, Berndt A, Sundberg JP, Uitto J. Mutant Enpp1asj mice as a model for generalized arterial calcification of infancy. Dis Model Mech. 2013 Sep;6(5):1227-35.
Mesken J, Iltzsche A, Mulac D, Langer K. Modifying plasmid-loaded HSA-nanoparticles with cell penetrating peptides - Cellular uptake and enhanced gene delivery. Int J Pharm. 2017 Apr 30;522(1-2):198-209
Michaelis M, Langer K, Arnold S, Doerr HW, Kreuter J, Cinatl J Jr. Pharmacological activity of DTPA linked to protein-based drug carrier systems. Biochem Biophys Res Commun. 2004 Oct 29;323(4):1236-40.
Najjar DM, Cohen EJ, Rapuano CJ, Laibson PR. EDTA chelation for calcific band keratopathy: results and long-term follow-up. Am J Ophthalmol. 2004 Jun;137(6):1056-64.
Okawa A, Nakamura I, Goto S, Moriya H, Nakamura Y, Ikegawa S. Mutation in Npps in a mouse model of ossification of the posterior longitudinal ligament of the spine. Nat Genet. 1998 Jul;19(3):271-3
Paul H, Reginato AJ, Schumacher HR. Alizarin red S staining as a screening test to detect calcium compounds in synovial fluid. Arthritis Rheum. 1983 Feb;26(2):191-200.
Saito E, Suarez-Gonzalez D, Rao RR, Stegemann JP, Murphy WL, Hollister SJ. Use of micro-computed tomography to nondestructively characterize biomineral coatings on solid freeform fabricated poly (L-lactic acid) and poly ((ε-caprolactone) scaffolds in vitro and in vivo. Tissue Eng Part C Methods. 2013 Jul;19(7):507-17.
Sarkar BC, Chauhan UP. A new method for determining micro quantities of calcium in biological materials. Anal Biochem. 1967 Jul;20(1):155-66.
Singh SR, Grossniklaus HE, Kang SJ, Edelhauser HF, Ambati BK, Kompella UB. Intravenous transferrin, RGD peptide and dual-targeted nanoparticles enhance anti-VEGF intraceptor gene delivery to laser-induced CNV. Gene Ther. 2009 May;16(5):645-59
Spraul CW, Lang GE, Grossniklaus HE, Lang GK. Histologic and morphometric analysis of the choroid, Bruch's membrane, and retinal pigment epithelium in postmortem eyes with age-related macular degeneration and histologic examination of surgically excised choroidal neovascular membranes. Surv Ophthalmol. 1999 Oct;44 Suppl 1:S10-32
Szmacinski H, Hegde K, Zeng HH, Eslami K, Puche AC, Lengyel I, Thompson RB. Imaging hydroxyapatite in sub-retinal pigment epithelial deposits by fluorescence lifetime imaging microscopy with tetracycline staining. J Biomed Opt. 2020 Apr;25(4):1-11
Tan ACS, Pilgrim MG, Fearn S, Bertazzo S, Tsolaki E, Morrell AP, Li M, Messinger JD, Dolz-Marco R, Lei J, Nittala MG, Sadda SR, Lengyel I, Freund KB, Curcio CA. Calcified nodules in retinal drusen are associated with disease progression in age-related macular degeneration. Sci Transl Med. 2018 Nov 7;10(466):eaat4544.
Thompson RB, Reffatto V, Bundy JG, Kortvely E, Flinn JM, Lanzirotti A, Jones EA, McPhail DS, Fearn S, Boldt K, Ueffing M, Ratu SG, Pauleikhoff L, Bird AC, Lengyel I. Identification of hydroxyapatite spherules provides new insight into subretinal pigment epithelial deposit formation in the aging eye. Proc Natl Acad Sci U S A. 2015 Feb 3;112(5):1565-70.
Tripathy et al. Stat Pearls (Internet), Aug 2021[ Shukla UV, Tripathy K. Diabetic Retinopathy. [Updated 2023 Aug 25]. In: StatPearls [Internet]. Treasure Island (FL): StatPearls Publishing; 2024 Jan-. Available from: https://www.ncbi.nlm.nih.gov/books/NBK560805/].
van der Schaft TL, Mooy CM, de Bruijn WC, Oron FG, Mulder PG, de Jong PT. Histologic features of the early stages of age-related macular degeneration. A statistical analysis. Ophthalmology. 1992 Feb;99(2):278-86
Weber C, Kreuter J, Langer K. Desolvation process and surface characteristics of HSA-nanoparticles. Int J Pharm. 2000 Mar 10;196(2):197-200
Weber C, Reiss S, Langer K. Preparation of surface modified protein nanoparticles by introduction of sulfhydryl groups. Int J Pharm. 2000 Dec 15;211(1-2):67-78 (=Weber et al., 2000a)
Yamaguchi A, Rajput AR, Ohzeki R, Kambara T. Determination of Ethylenediaminetetraacetic Acid and Nitrilotriacetic Acid as Their Iron(III)-Complexes by Reversed Phase High Performance Liquid Chromatography. Bull. Chem. Soc. Jpn. 1983;56(9):2621-2623

## Claims

1. A nanoparticle comprising a scaffold comprising a biodegradable material, an antibody, that is able to target a component of a Bruch's membrane, and an anti-calcifying agent for use in a prophylaxis or in a treatment of a pathological change of Bruch's membrane and/or an adjacent tissue, including a retinal pigment epithelium and a choroid of an eye.

2. The nanoparticle for use of claim 1, wherein the pathological change is a calcification of Bruch's membrane and/or the adjacent tissue, including the retinal pigment epithelium and the choroid of the eye.

3. The nanoparticle for use of any one of the preceding claims, wherein the biodegradable material is human serum albumin.

4. The nanoparticle for use of any one of the preceding claims, wherein the antibody is an anti-elastin antibody or an anti-vitronectin antibody and wherein the antibody is preferably covalently linked to the nanoparticle.

5. The nanoparticle for use of any one of the preceding claims, wherein the antibody is a Fab fragment.

6. The nanoparticle for use of any one of the preceding claims, wherein the anti-calcifying agent is a chelator, preferably diethylenetriaminepentaacetic acid (DTPA) or ethylenediaminetetraacetic acid (EDTA), inorganic pyrophosphate (PPi), or sodium thiosulfate.

7. The nanoparticle for use of any one of the preceding claims, wherein the nanoparticle has a negative surface charge.

8. The nanoparticle for use of any one of the preceding claims, wherein the nanoparticle comprises the scaffold comprising human serum albumin, the anti-elastin antibody preferably covalently linked to the nanoparticle, and DTPA preferably covalently linked to the nanoparticle.

9. A pharmaceutical composition for use of any of the claims 1 to 8, comprising the nanoparticle of any one of the preceding claims and pharmaceutical acceptable excipients for use in a prophylaxis or in a treatment of a pathological change of Bruch's membrane and/or adjacent tissues, including a retinal pigment epithelium and a choroid of an eye.

10. The pharmaceutical composition for use of claim 9, wherein the pathological change is a calcification of Bruch's membrane and/or an adjacent tissue, including a retinal pigment epithelium and a choroid of an eye.

11. The nanoparticle for use of any one of claims 1 to 8 or the pharmaceutical composition for use of claim 9 for use in the prophylaxis or treatment of a disease, wherein the calcification of Bruch's membrane and/or adjacent tissues, including the retinal pigment epithelium and the choroid of an eye is a symptom of the disease.

12. The nanoparticle for use or the pharmaceutical composition for use of claim 11, wherein the disease is selected from the group consisting of Pseudoxanthoma elasticum, Angioid Streaks, peau d'orange fundus, Beta-Thalassemia, Sickle Cell disease, Juvenile Paget's disease, Hyperphosphatemic familial tumoral calcinosis (HFTC), ENPP1 deficiency, Idiopathic Sclerochoroidal calcification, Choroidal calcification associated to chondrocalcinosis (pseudogout), Sclerochoroidal Calcification with optic nerve calcification in primary hyperparathyroidism or parathyroid adenoma, Genetic renal tubulopathies: like Bartter syndrome, and Gitelman syndrome, Aplasia cutis congenita and oculoectodermal syndrome, Cytomegalovirus Retinitis, Age-related macular degeneration, Refractile drusen (a feature of AMD), and Optic Nerve Head Drusen.

13. The nanoparticle for use of any one of claims 1 to 8 or 11 or 12 or the pharmaceutical composition for use of any one of claims 9 to 12, wherein the nanoparticle or the pharmaceutical composition is administered intravenously, intravitreally, subconjunctivally, subtenonly, retrobulbarly, posteriorly, juxtasclerally, suprachoroideally, subretinally, or topically as eye drops.

## Patentansprüche

1. Ein Nanopartikel, umfassend ein Gerüst umfassend ein biologisch abbaubares Material, einen Antikörper, der in der Lage ist, an eine Komponente der Bruch´schen Membran zu binden, und ein Antikalzifikationsmittel zur Verwendung bei der Prophylaxe oder Behandlung einer pathologischen Veränderung der Bruch´schen Membran und/oder eines angrenzenden Gewebes, einschließlich des retinalen Pigmentepithels und der Aderhaut eines Auges.

2. Das Nanopartikel zur Verwendung nach Anspruch 1, wobei die pathologische Veränderung eine Verkalkung der Bruch´schen Membran und/oder des angrenzenden Gewebes ist, einschließlich des retinalen Pigmentepithels und der Aderhaut des Auges.

3. Der Nanopartikel zur Verwendung nach einem der vorstehenden Ansprüche, wobei das biologisch abbaubare Material Human-Serumalbumin ist.

4. Der Nanopartikel zur Verwendung nach einem der vorstehenden Ansprüche, wobei der Antikörper ein Anti-Elastin-Antikörper oder ein Anti-Vitronectin-Antikörper ist und wobei der Antikörper vorzugsweise kovalent an das Nanopartikel gebunden ist.

5. Der Nanopartikel zur Verwendung nach einem der vorstehenden Ansprüche, wobei der Antikörper ein Fab-Fragment ist.

6. Der Nanopartikel zur Verwendung nach einem der vorstehenden Ansprüche, wobei das Antikalzifikationsmittel ein Chelatbildner ist, vorzugsweise Diethylentriaminpentaessigsäure (DTPA) oder Ethylendiamintetraessigsäure (EDTA), anorganisches Pyrophosphat (PPi) oder Natriumthiosulfat.

7. Der Nanopartikel zur Verwendung nach einem der vorstehenden Ansprüche, wobei der Nanopartikel eine negative Oberflächenladung aufweist.

8. Der Nanopartikel zur Verwendung nach einem der vorstehenden Ansprüche, wobei der Nanopartikel das Gerüst umfassend humanes Serumalbumin, den vorzugsweise kovalent an den Nanopartikel gebundenen Anti-Elastin-Antikörper und das vorzugsweise kovalent an den Nanopartikel gebundene DTPA umfasst.

9. Eine Pharmazeutische Zusammensetzung zur Verwendung nach einem der Ansprüche 1 bis 8, umfassend den Nanopartikel gemäß einem der vorstehenden Ansprüche und pharmazeutisch verträgliche Hilfsstoffe zur Verwendung bei der Prophylaxe oder Behandlung einer pathologischen Veränderung der Bruch´schen Membran und/oder angrenzender Gewebe, einschließlich des retinalen Pigmentepithels und der Aderhaut eines Auges.

10. Die pharmazeutische Zusammensetzung zur Verwendung nach Anspruch 9, wobei die pathologische Veränderung eine Verkalkung der Bruch´schen Membran und/oder eines angrenzenden Gewebes ist, einschließlich des retinalen Pigmentepithels und der Aderhaut eines Auges.

11. Das Nanopartikel zur Verwendung gemäß einem der Ansprüche 1 bis 8 oder die pharmazeutische Zusammensetzung zur Verwendung gemäß Anspruch 9 zur Verwendung bei der Prophylaxe oder Behandlung einer Erkrankung, wobei die Verkalkung der Bruch´schen Membran und/oder angrenzender Gewebe, einschließlich des retinalen Pigmentepithels und der Aderhaut eines Auges, ein Symptom der Erkrankung ist.

12. Das Nanopartikel zur Verwendung oder die pharmazeutische Zusammensetzung zur Verwendung nach Anspruch 11, wobei die Erkrankung ausgewählt is, aus der Gruppe bestehend aus Pseudoxanthoma elasticum, angioide Streifen, Orangenhaut-Fundus, Beta-Thalassämie, Sichelzellenanämie, juveniler Paget-Krankheit, hyperphosphatämische familiäre tumorale Kalzinose (HFTC), ENPP1-Mangel, idiopathische sklero-choroidale Kalzifikation, choroidale Kalzifikation assoziiert mit Chondrokalzinose (Pseudogicht) , sklero-choroidale Kalzifikation mit Sehnervenkalzifikation bei primärem Hyperparathyreoidismus oder Parathyreoidadenom, genetische Nierentubulopathien wie das Bartter-Syndrom und das Gitelman-Syndrom, Aplasia cutis congenita und das oculoektodermale Syndrom, Cytomegalievirus-Retinitis, altersbedingte Makuladegeneration, refraktile Drusen (ein Merkmal der AMD) und Drusen am Sehnervenkopf.

13. Das Nanopartikel zur Verwendung nach einem der Ansprüche 1 bis 8 oder 11 oder 12 oder die pharmazeutische Zusammensetzung zur Verwendung nach einem der Ansprüche 9 bis 12, wobei das Nanopartikel oder die pharmazeutische Zusammensetzung intravenös, intravitreal, subkonjunktival, subtenonisch, retrobulbär, posterior, juxtaskleral, suprachoroidal, subretinal oder topisch als Augentropfen verabreicht wird.

## Revendications

1. Une nanoparticule comprenant une structure de support constituée d'un matériau biodégradable, un anticorps capable de cibler un composant de la membrane de Bruch, et un agent anti-calcification, destinée à être utilisée dans la prophylaxie ou le traitement d'une altération pathologique de la membrane de Bruch et/ou d'un tissu adjacent, notamment l'épithélium pigmentaire rétinien et la choroïde de l'œil.

2. La nanoparticule destinée à être utilisée selon la revendication 1, dans laquelle l'altération pathologique est une calcification de la membrane de Bruch et/ou du tissu adjacent, y compris l'épithélium pigmentaire rétinien et la choroïde de l'œil.

3. La nanoparticule destinée à être utilisée selon l'une quelconque des revendications précédentes, dans laquelle le matériau biodégradable est l'albumine sérique humaine.

4. La nanoparticule destinée à l'usage selon l'une quelconque des revendications précédentes, dans laquelle l'anticorps est un anticorps anti-élastine ou un anticorps anti-vitronectine et dans laquelle l'anticorps est de préférence lié de manière covalente à la nanoparticule.

5. La nanoparticule destinée à l'usage selon l'une quelconque des revendications précédentes, dans laquelle l'anticorps est un fragment Fab.

6. La nanoparticule destinée à être utilisée selon l'une quelconque des revendications précédentes, dans laquelle l'agent anti-calcification est un chélateur, de préférence l'acide diéthylènetriaminepentaacétique (DTPA) ou l'acide éthylènediaminetétraacétique (EDTA), un pyrophosphate inorganique (PPi) ou du thiosulfate de sodium.

7. La nanoparticule destinée à être utilisée selon l'une quelconque des revendications précédentes, dans laquelle la nanoparticule présente une charge superficielle négative.

8. La nanoparticule destinée à être utilisée selon l'une quelconque des revendications précédentes, **caractérisée en ce que** la nanoparticule comprend une structure de support constituée d'albumine sérique humaine, l'anticorps anti-élastine lié de préférence de manière covalente à la nanoparticule, et du DTPA lié de préférence de manière covalente à la nanoparticule.

9. Une composition pharmaceutique destinée à être utilisée selon l'une quelconque des revendications 1 à 8, comprenant la nanoparticule selon l'une quelconque des revendications précédentes et des excipients pharmaceutiquement acceptables, destinée à être utilisée dans la prophylaxie ou le traitement d'une altération pathologique de la membrane de Bruch et/ou des tissus adjacents, y compris l'épithélium pigmentaire rétinien et la choroïde de l'œil.

10. La composition pharmaceutique destinée à être utilisée selon la revendication 9, dans laquelle l'altération pathologique est une calcification de la membrane de Bruch et/ou d'un tissu adjacent, notamment l'épithélium pigmentaire rétinien et la choroïde de l'œil.

11. La nanoparticule selon l'une quelconque des revendications 1 à 8 ou la composition pharmaceutique selon la revendication 9, destinée à être utilisée dans la prophylaxie ou le traitement d'une maladie, dans laquelle la calcification de la membrane de Bruch et/ou des tissus adjacents, y compris l'épithélium pigmentaire rétinien et la choroïde de l'œil, est un symptôme de ladite maladie.

12. La nanoparticule destinée à être utilisée ou la composition pharmaceutique destinée à être utilisée selon la revendication 11, dans laquelle la maladie est choisie parmi le groupe constitué du pseudoxanthome élastique, des stries angioïdes, du fond d'œil en peau d'orange, de la bêta-thalassémie, de la drépanocytose, de la maladie de Paget juvénile, de la calcinose tumorale familiale hyperphosphatémique (HFTC), le déficit en ENPP1, la calcification sclérochoroïdienne idiopathique, la calcification choroïdienne associée à la chondrocalcinose (pseudogoutte), la calcification sclérochoroïdienne avec calcification du nerf optique dans l'hyperparathyroïdie primaire ou l'adénome parathyroïdien, les tubulopathies rénales génétiques : telles que le syndrome de Bartter et le syndrome de Gitelman, l'aplasie cutanée congénitale et le syndrome oculo-ectodermique, la rétinite à cytomégalovirus, la dégénérescence maculaire liée à l'âge, les drusen réfractiles (une caractéristique de la DMLA) et les drusen de la tête du nerf optique.

13. La nanoparticule destinée à être utilisée selon l'une quelconque des revendications 1 à 8, 11 ou 12, ou la composition pharmaceutique destinée à être utilisée selon l'une quelconque des revendications 9 à 12, dans laquelle la nanoparticule ou la composition pharmaceutique est administrée par voie intraveineuse, intravitréenne, sous-conjonctivale, sous-ténonienne, rétrobulbaire, postérieure, juxtasclérale, suprachoroïdienne, sous-rétinienne ou par voie topique sous forme de collyre.
